# EUROPEAN PATENT SPECIFICATION

(11) **EP 1 987 025 B1**
(45) Date of publication and mention of the grant of the patent: **16.09.2009**
(21) Application number: 07705605.9
(22) Date of filing: 05.02.2007
(51) Int. Cl.: C07D 413/04, C07D 413/14, A61K 31/553, A61P 31/04

(54) **BENZOXAZINONE AND BENZOXAZEPINONE OXAZOLIDINONES AS ANTIBACTERIAL AGENTS**
BENZOXAZINON- UND BENZOXAZEPINONOXAZOLIDINONE ALS ANTIBAKTERIELLE MITTEL
OXAZOLIDINONES BENZOXAZINONE ET BENZOXAZÉPINONE EN TANT QU'AGENTS ANTIBACTÉRIENS

(30) Priority: 14.02.2006 US 773071 P
(43) Date of publication of application: 05.11.2008
(73) Proprietor: Pfizer Products Inc., Groton, CT 06340 (US)
(72) Inventor: GAO, Hongwu, Woodside, New York 11377 (US); GORDEEV, Mikhail, Castro Valley, CA 94552 (US); LUEHR, Gary, Wayne, Hayward, CA 94542 (US); VENKATA NAGENDRA JOSYULA, Vara, Prasad, Ann Arbor, Michigan 48105 (US); RENSLO, Adam, Robert, Oakland, CA 94618 (US)
(74) Representative: Kingsbury, Oliver William
(86) International application number: PCT/IB2007/000377
(87) International publication number: WO 2007/093904

(56) References cited:
- WO-A-00/29409
- WO-A-20/05082897
- DE-A1- 19 802 239

## Description

### FIELD OF INVENTION

The present invention relates to novel benzoxazinone and benzoxazepinone derivatives of oxazolidinones, pharmaceutical compositions thereof, methods for their use, and methods for preparation. These compounds have potent activities against gram-positive bacteria.

### BACKGROUND OF THE INVENTION

Antibacterial resistance is a global clinical and public health problem that has emerged with alarming rapidity in recent years and undoubtedly will increase in the near future. Resistance is a problem in the community as well as in health care settings, where transmission of bacteria is greatly amplified. Because multiple drug resistance is a growing problem, physicians are now confronted with infections for which there is no effective therapy. As result, structurally novel antibacterials with a new mode of action have become increasingly important in the treatment of bacterial infections.

Among newer antibacterial agents, oxazolidinone compounds are the most recent synthetic class of antimicrobials active against a number of pathogenic microorganisms. This invention provides novel benzoxazinone and benzoxazepinone derivatives of oxazolidinones, and their preparation.

### INFORMATION DISCLOSURE

PCT publication WO 9937641 discloses 1,4-benzoxazin-3-one and 1,4-benzothiazin-3-one oxazolidinone derivatives as antibacterial agents

PCT publication WO 9940094 discloses 1,3-benzoxazin-2-one and 1,3-benzothiazin-2-thione intermediates used in the preparation of azole-containing tricyclic oxazolidinones useful as antibacterials.

PCT publication WO 9964416 discloses oxazolidinone derivatives.

PCT publication WO 9964417 discloses oxazolidinone derivatives.

PCT publication WO 200021960 discloses heterocyclyl aminomethyl oxazolidinone derivatives.

PCT publication WO 200029409 discloses oxazolidinone derivatives.

PCT publication WO 200181350 discloses oxazolidinone derivatives and their salts or *in vivo* hydrolysable esters.

PCT publication WO 200281470 discloses oxazolidinone compounds and their salts or *in vivo* hydrolysable esters.

PCT publication WO 2003035648 discloses aryl substituted oxazolidinones, their salts or esters.

PCT publication WO 2005082897 discloses amidoxime and amidine oxazolidinone antibacterial agents.

U.S. Patent 5,171,851 discloses 1,3-benzoxazin-2-one, 1,3-benzothiazin-2-one and 1,3-benzothiazin-2-thione derivatives as cardiotonic agents.

DE 19604223, DE 19805117, DE 19901306, DE 19905278, DE 19962924, EP 738726, and EP 785201 disclose oxazolidinone derivatives.

### SUMMARY OF THE INVENTION

The present invention provides a compound of formula I 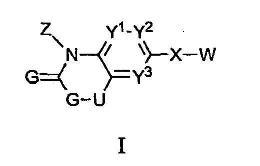 or a pharmaceutically acceptable salt thereof wherein:
X is a structure of the following formula i, ii, iii, or iv 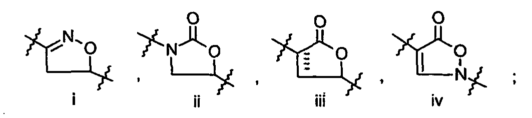
G is O, or S;
U is -(CR³R⁴)ₙ-, or CF₂;
W is
   (a) CH₂NHC(=O)R¹,
   (b) CH₂NHC(=S)R¹,
   (c) CH₂NH-het,
   (d) CH₂O-het,
   (e) CH₂S-het,
   (f) CH₂het,
   (g) C(=O)NHR², or
   (h) CH₂OH;
Y¹, Y² and Y³ are independently
   (a) CH,
   (b) N,
   (c) N⁺-O⁻, or
   (d) CF;
Z is C₁₋₄alkyl, optionally substituted with 1-3 fuloro;
R¹ is
   (a) NH₂,
   (b) NHC₁₋₆alkyl,
   (c) C₁₋₆alkyl,
   (b) C₂₋₆alkenyl,
   (c) C₃₋₇cycloalkyl,
   (e) (CH₂)ⱼC(=O)C₁₋₄alkyl,
   (f) OC₁₋₆alkyl,
   (g) SC₁₋₆alkyl, or
   (h) (CH₂)ⱼC₃₋₇cycloalkyl;
R² is
   (a) H,
   (b) C₁₋₆alkyl,
   (c) C₂₋₆alkenyl,
   (d) C₃₋₇cycloalkyl, or
   (e) -OC₁₋₄alkyl;
R³ and R⁴ are independently
   (a) H
   (b) C₁₋₆ alkyl, or
   (c) R³ and R⁴ taken together with the carbon atom to which they attach form C₃₋₇cycloalkyl;
het is a five- (5) or six- (6) membered heterocyclic ring having 1-4 heteroatoms selected from the group consisting of oxygen, sulfur, and nitrogen within the ring, wherein each carbon atom in het is optionally substituted with C₁₋₆alkyl, C₂₋₆alkenyl, C₂₋₆alkynyl, C₃₋₇cycloalkyl, halo, OR⁵, SR⁵, CN, NO₂, NR⁵R⁶, oxo, CF₃, OCF₃, C(=O)C₁₋₄alkyl, OC(=O)C₁₋₄alkyl, C(=O)OR⁵, or S(O)ₘC₁₋₄alkyl; at each occurrence, C₁₋₆alkyl, C₂₋₆alkenyl, C₂₋₆alkynyl, or C₃₋₇cycloalkyl is optionally substituted with 1-3 halo, OR⁵, SR⁵, CN, N₃, NO₂, NR⁵R⁶, C(=O)C₁₋₄alkyl, OC(=O)C₁₋₄alkyl, C(=O)OC₁₋₄alkyl, or S(O)ₘC₁₋₄alkyl, wherein R⁵ and R⁶ are independently H, or C₁₋₄alkyl, optionally substituted with OH, phenyl, or OC₁₋₄alkyl;
each n is independently 1 or 2;
m is 0, 1, or 2; and
each j is independently 0-4.

In another aspect, the present invention also provides:
a pharmaceutical composition which comprises a pharmaceutically acceptable carrier and an effective amount of a compound of formula I,
a compound of formula I or a pharmaceutically acceptable salt thereof for treating gram-positive microbial infections in a mammal by administering to the subject in need a therapeutically effective amount of and
a use of a compound of formula I or a pharmaceutically acceptable salt thereof to prepare a medicament for treating gram-positive microbial infections.

The invention may also provide novel intermediates and novel processes that are useful for preparing compounds of formula I.

### DETAILED DESCRIPTION OF THE INVENTION

Unless otherwise stated, the following terms used in the specification and claims have the meanings given below:

The carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, C₁₋₇ alkyl refers to alkyl of one to seven carbon atoms, inclusive.

The term alkyl, or alkenyl, etc. refer to both straight and branched groups, but reference to an individual radical such as "propyl" embraces only the straight chain radical, a branched chain isomer such as "isopropyl" being specifically referred to.

The term "C₃₋₇cycloalkyl" refers to a cyclic saturated monovalent hydrocarbon group of three to seven carbon atoms, e.g., cyclopropyl, cyclohexyl, and the like.

The term "halo" refers to fluoro (F), chloro (Cl), bromo (Br), or iodo (I).

The term "het" is a five- (5) or six- (6) membered heterocyclic ring having 1-4 heteroatoms selected from the group consisting of oxygen, sulfur, and nitrogen within the ring. An examples of het includes, but are not limited to, pyrrole, imidazole, pyrazole, 1,2,3-triazole, 1,3,4-triazole, oxazole, thiazole, isoxazole, isothiazole, 1,3,4-oxadiazole, 1,3,4-thiadiazole, 1,2,3-thiadiazole, tetrazole, pyridine, pyrazine, pyrimidine, pyridazine, indolizine, isoindole, indole, dihydroindole, indazole, purine, quinolizine, isoquinoline, quinoline, phthalazine, quinoxaline, quinazoline, cinnoline, pteridine, carbazole, carboline, phenanthridine, acridine, phenanthroline, isothiazole, phenazine, isoxazole, isoxazolinone, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiadiazole, tetrazole, thiazolidine, thiophene, benzo[b]thiophene, morpholine, thiomorpholine, (also referred to as thiamorpholine,), piperidine, pyrrolidine, tetrahydrofuran, or the like. Another example of het includes, but are not limited to, pyridine, thiophene, furan, pyrazole, pyrimidine, 2-pyridyl, 3-pyridyl, 4-pyridyl, 2-pyrimidinyl, 4-pyrimidinyl, 5-pyrimidinyl, 3-pyridazinyl, 4-pyridazinyl, 3-pyrazinyl, 4-oxo-2-imidazolyl, 2-imidazolyl, 4-imidazolyl, 3-isoxaz-olyl, 4-is-oxaz-olyl, 5-isoxaz-olyl, 3-pyrazolyl, 4-pyrazolyl, 5-pyrazolyl, 2-oxazolyl, 4-oxazolyl, 4-oxo-2-oxazolyl, 5-oxazolyl, 1,2,3-oxathiazole, 1,2,3-oxadiazole, 1,2,4-oxadiazole, 1,2,5-oxadiazole, 1,3,4-oxadiazole, 2-thiazolyl, 4-thiazolyl, 5-thiazolyl, 3-isothiazole, 4-isothiazole, 5-isothiazole, 2-furanyl, 3-furanyl, 2-thienyl, 3-thienyl, 2-pyrrolyl, 3-pyrrolyl, 3-isopyrrolyl, 4-isopyrrolyl, 5-isopyrrolyl, 1,2,3,-oxathiazole-1-oxide, 1,2,4-oxadiazol-3-yl, 1,2,4-oxadiazol-5-yl, 5-oxo-1,2,4-oxadiazol-3-yl, 1,2,4-thiadiazol-3-yl, 1,2,5-thiadiazol-3-yl, 1,2,4-thiadiazol-5-yl, 3-oxo-1,2,4-thiadiazol-5-yl, 1,3,4-thiadiazol-5-yl, 2-oxo-1,3,4-thiadiazol-5-yl, 1,2,3-triazole-1-yl, 1,2,4-triazol-3-yl, 1,2,4-triazol-5-yl, tetrazole-1-yl, 1,2,3,4-tetrazol-5-yl, 5-oxazolyl, 3-isothiazolyl, 4-isothiazolyl and 5-isothiazolyl, 1,3,4,-oxadiazole, 4-oxo-2-thiazolinyl, or 5-methyl-1,3,4-thiadiazol-2-yl, thiazoledione, 1,2,3,4-thiatriazole, or 1,2,4-dithiazolone.

The term "a pharmaceutically acceptable salt" of a compound means a salt that is pharmaceutically acceptable and that possesses the desired pharmacological activity of the parent compound. Such salts include:
(1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or
(2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like.

The term "pharmaceutically acceptable carrier" means a carrier that is useful in preparing a pharmaceutical composition that is generally safe, non-toxic and neither biologically nor otherwise undesirable, and includes a carrier that is acceptable for veterinary use as well as human pharmaceutical use. "A pharmaceutically acceptable carrier" as used in the specification and claims includes both one and more than one such carrier.

The term "mammal" refers to human or warm-blooded animals including livestock and companion animals.

The term "optional" or "optionally" means that the subsequently described event or circumstance may, but need not, occur, and that the description includes instances where the event or circumstance occurs and instances in which it does not.

Compounds that have the same molecular formula but differ in the nature or sequence of bonding of their atoms or the arrangement of their atoms in space are termed "isomers". Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this invention may possess one or more asymmetric centers; such compounds can therefore be produced as individual (R)- or (S)- stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 1992).

The term "treating" or "treatment" of a disease includes: (1) preventing the disease, i.e. causing the clinical symptoms of the disease not to develop in a mammal that may be exposed to or predisposed to the disease but does not yet experience or display symptoms of the disease; (2) inhibiting the disease, i.e., arresting or reducing the development of the disease or its clinical symptoms; or (3) relieving the disease, i.e., causing regression of the disease or its clinical symptoms.

The term "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

The term "leaving group" has the meaning conventionally associated with it in synthetic organic chemistry i.e., an atom or group capable of being displaced by a nucleophile and includes halogen, alkylsulfonyloxy, ester, or amino such as chloro, bromo, iodo, mesyloxy, tosyloxy, trifluorosulfonyloxy, methoxy, N,O-dimethylhydroxyl-amino, and the like.

The compounds of the present invention are generally named according to the IUPAC or CAS nomenclature system.

Abbreviations which are well known to one of ordinary skill in the art may be used (e.g. "Ph" for phenyl, "Me" for methyl, "Et" for ethyl, "h" for an hour or hours and "rt" for room temperature).

Specific and preferred values listed below for radicals, substituents, and ranges, are for illustration only; they do not exclude other defined values or other values within defined ranges for the radicals and substituents.

Specifically, alkyl denotes both straight and branched groups; but reference to an individual radical such as "propyl" embraces only the straight chain radical, a branched chain isomer such as "isopropyl" being specifically referred to.

Specifically, alkyl is methyl, ethyl, propyl, isopropyl, butyl, iso-butyl, sec-butyl, and their isomeric forms thereof.

Specifically, alkyl is methyl, ethyl, propyl, isopropyl, butyl, iso-butyl, sec-butyl, and their isomeric forms thereof.

Specifically, cycloalkyl is cyclopropyl, cyclobutyl, cyclopentyl, cyclohexyl, and their isomeric forms thereof.

Specifically, halo is fluoro (F), chloro (Cl).

Specifically W is CH₂NHC(=O)R¹.

Specifically, R¹ is C₁₋₄alkyl, optionally substituted with one, two or three fluoro (F), or chloro (Cl).

Specifically, R¹ is OC₁₋₄alkyl, optionally substituted with one, two or three fluoro (F), or chloro (Cl).

Specifically, R¹ is CH₃, CH₂CH₂, or OCH₃.

Specifically, W is CH₂het.

Specifically, W is 1,2,3-triazole-1-yl methyl, or tetrazole-1-yl methyl.

Specifically, W is C(=O)NHR².

Specifically, R² is C₁₋₄alkyl, or OC₁₋₄alkyl.

Specifically, R² is H, CH₃, or SOCH₃.

Specifically, Y¹, Y², or Y³ is CH.

Specifically, Y¹ is CF, Y² and Y³ are CH.

Specifically, Z is CH₃.

Specifically, U is CH₂, or CH₂CH₂.

Specifically, X is a structure of formula ii

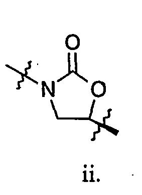

Specifically, the formula I of the present invention is a compound of formula II 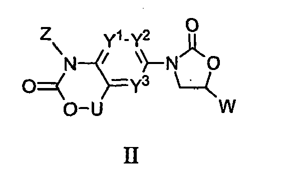 wherein U is CH₂, or CH₂CH₂; W is CH₂NHC(=O)R¹, CH₂het, or C(=O)NHR²; Y¹, Y² and Y³ are independently CH, or CF; R¹ is C₁₋₄alkyl, or OC₁₋₄alkyl; R² is H, C₁₋₄alkyl, or -OC₁₋₄alkyl; and Z is CH₃.

Examples of the present invention include:
(1) (S)-N-((3-(1-methyl-2-oxo-2,4-dihydro-1H-benzo[d][1,3]oxazin-6-yl)-2-oxooxazolidin-5-yl)methyl)acetamide,
(2) (*S*)-*N*-((3-(1-methyl-2-oxo-2,4-dihydro-1H-benzo[*d*][1,3]oxazin-6-yl)-2-oxooxazolidin-5-yl)methyl)propionamide,
(3) (*S*)-methyl (3-(1-methyl-2-oxo-2,4-dihydro-1H-benzo[d][1,3]oxazin-6-yl)-2-oxooxazolidin-5-yl)methylcarbamate,
(4) (*R*)-3-(1-methyl-2-oxo-2,4-dihydro-1H-benzo[d][1,3]oxazin-6-yl)-2-oxooxazolidine-5-carboxamide,
(5) (*R*)-*N*-methyl-3-(1-methyl-2-oxo-2,4-dihydro-1H-benzo[d][1,3]oxazin-6-yl)-2 oxooxazolidine-5-carboxamide,
(6) (*S*)-fuoromethyl (3-(1-methyl-2-oxo-2,4-dihydro-1H-benzo[d][1,3]oxazin-6-yl)-2-oxooxazolidin-5-yl)methylcarbamate,
(7) (*R*)-6-(5-((1H-1,2,3-triazol-1-yl)methyl)-2-oxooxazolidin-3-yl)-1-methyl-1H-benzo[d][1,3]oxazin-2(4H)-one,
(8) (*R*)-3-(8-fluoro-1-methyl-2-oxo-2,4-dihydro-1H-benzo[d][1,3]oxazin-6-yl)-2-oxooxazolidine-5-carboxamide,
(9) (*R*)-3-(8-fluoro-1-methyl-2-oxo-2,4-dihydro-1H-benzo[d][1,3]oxazin-6-yl)-N-methyl-2-oxooxazolidine-5-carboxamide,
(10) (*S*)-*N*-((3-(8-fluoro-1-methyl-2-oxo-2,4-dihydro-1H-benzo[d][1,3]oxazin-6-yl)-2-oxooxazolidin-5-yl)methyl)acetamide,
(11) (*S*)-*N*-((3-(8-fluoro-1-methyl-2-oxo-2,4-dihydro-1H-benzo[d][1,3]oxazin-6-yl)-2-oxooxazolidin-5-yl)methyl)propionamide,
(12) (*S*)-methyl (3-(8-fluoro-1-methyl-2-oxo-2,4-dihydro-1H-benzo[d][1,3]oxazin-6-yl)-2-oxooxazolidin-5-yl)methylcarbamate,
(13) (*S*)-*N*-((3-(5,8-difluoro-1-methyl-2-oxo-2,4-dihydro-1H-benzo[d][1,3]oxazin-6-yl)-2-oxooxazolidin-5-yl)methyl)acetamide,
(14) (*S*)-methyl (3-(5,8-difluoro-1-methyl-2-oxo-2,4-dihydro-1H-benzo[d][1,3]oxazin-6-yl)-2-oxooxazolidin-5-yl)methylcarbamate,
(15) *N*-{[(5*S*)-3-(9-Fluoro-1-methyl-2-oxo-1,2,4,5-tetrahydro-3,1-benzoxazepin-7-yl)-2-oxo-1,3-oxazolidin-5-yl]methyl}acetamide,
(16) *N*-{[(5*S*)-3-(9-fluoro-1-methyl-2-oxo-1,2,4,5-tetrahydro-3,1-benzoxazepin-7-yl)-2-oxo-1,3-oxazolidin-5-yl]methyl}propanamide,
(17) methyl [(5*S*)-3-(9-fluoro-1-methyl-2-oxo-1,2,4,5-tetrahydro-3,1-benzoxazepin-7-yl)-2-oxo-1,3-oxazolidin-5-yl]methylcarbamate,
(18) (5*R*)-3-(1-methyl-2-oxo-1,2,4,5-tetrahydro-3,1-benzoxazepin-7-yl)-2-oxo-1,3-oxazolidine-5-carboxamide,
(19) (5*R*)-*N-*methyl-3-(1-methyl-2-oxo-1,2,4,5-tetrahydro-3,1-benzoxazepin-7-yl)-2-oxo-1,3-oxazolidine-5-carboxamide,
(20) *N*-{[(5*S*)-3-(1-methyl-2-oxo-1,2,4,5-tetrahydro-3,1-benzoxazepin-7-yl)-2-oxo-1,3-oxazolidin-5-yl]methyl} acetamide,
(21) *N*-{[(5*S*)-3-(1-methyl-2-oxo-1,2,4,5-tetrahydro-3,1-benzoxazepin-7-yl)-2-oxo-1,3-oxazolidin-5-yl]methyl}propanamide, or
(22) methyl [(5*S*)-3-(1-methyl-2-oxo-1,2,4,5-tetrahydro-3,1-benzoxazepin-7-yl)-2-oxo-1,3-oxazolidin-5-yl]methylcarbamate.

Compounds of this invention can be prepared in accordance with one or more of the Schemes discussed below. All of the starting materials are either commercially available or can be prepared by procedures that would be well known to one of ordinary skill in organic chemistry. The variables used in the Schemes are as defined below, or as in the summary of the invention or claims.

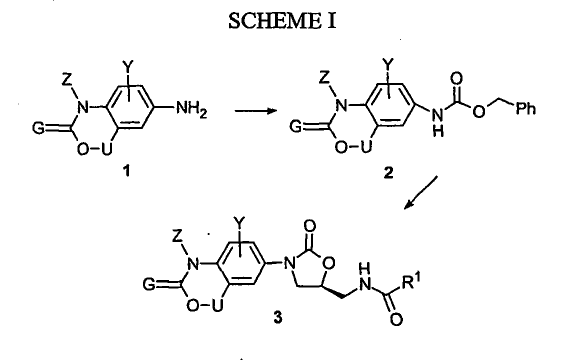

Scheme I illustrates construction of the oxazolidinone ring system bearing a acetylaminomethyl substituent at C-5 from an appropriately substututed 5-amino-1,3-benzoxazinone or 1,3-benzoxazepinone precursor **(1).** The aniline intermediate **(1)** is first converted to an aryl carbamate using standard procedures that are well known to those skilled in the art, for example using benzyl chloroformate and pyridine in dichloromethane. Formation of the oxazolidinone ring can then be accomplished using a multi-step procedure as described in Angewandte Chemie 2003, 42, 2010 (and references therein). Alternatively the conversion can be accomplished in one step using (*S*)-acetic acid 2-acetylamino-1-chloromethyl-ethyl ester. This reagent is prepared from (S)-epichlorohydrin in three steps (epoxide ring opening with benzaldehyde imine, imine hydrolysis, and peracylation with acetic anhydride) according to the procedure described in US Patent No. 6,833,453. The reaction is performed in the presence of an organic base such as lithium tert-butoxide, in a polar organic solvent such as dimethylformamide or acetonitrile, at temperatures of about 0 °C to 25 °C. The product may be used as collected or may first be purifed using conventional techniques such as preparative TLC or HPLC, chromatography, precipitation, crystallization and the like.

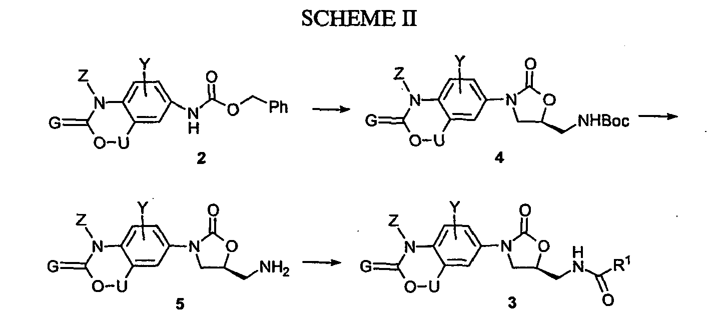

Scheme II illustrates the construction of the oxazolidinone ring when substitution other than acetylaminomethyl is desirable at C-5 of the oxazolidinone ring (i.e., R^{'} = alkyl, or O-alkyl, etc.). In this case, the aryl carbamate **(2)** is reacted with (S)-(3-chloro-2-hydroxypropyl)-carbamic acid *tert*-butyl ester (prepared as described in US Patent No. 6,833,453) using conditions analogous to those described for Scheme I, step 2. The product of this reaction is intermediate **(4)** bearing a tert-butyl carbamate (Boc protected amine) at the C-5 position. Intermediate **(4)** is deprotected by treatment with acids such as hydrochloric acid or trifluoric acid. If less harsh conditions are required, treatement with trimethylsilyltrifluoromethane sulfonate and 2,6-lutidine (as described by Ohfune, Y. and Sakaitani, M. J. Org. Chem. 1990, 55, 870-876) is also effective. Finally the amine can be converted to various amides using well-known coupling reactions (for example by treatment with acid chlorides) or to ureas and carbamates by reaction with isocyanates or alkyl chloroformates, respectively. The products may be used as collected or may first be purifed using conventional techniques such as preparative TLC or HPLC, chromatography, precipitation, crystallization and the like.

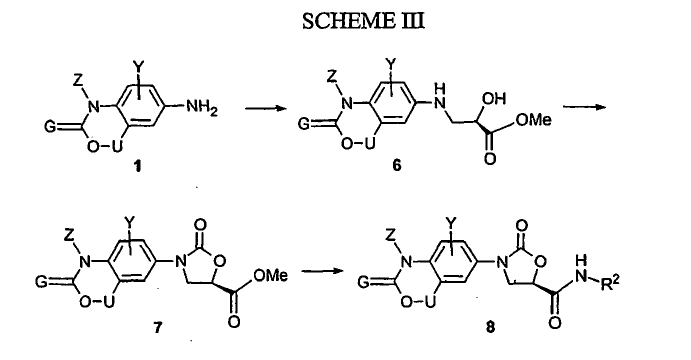

Carboxamide analogs may be prepared according to Scheme III. The substituted 5-amino-1,3-benzoxazinone or 1,3-benzoxazepinone precursor (**1**) is reacted with an alkyl (2*R*)-epoxypropanoate and a Lewis acid such as lithium triflate as described in US Patent 6,919,329. The amino alcohol (**6**) can then be ring closed to give the aryl oxazolidinones (**7** following conditions as described in Scheme **3**. Subsequent treatment of oxazolidinone ester **(7)** with ammonia or optionally substituted amines (R'NH₂) in a suitable solvent such as methanol or acetonitrile affords amides (**8**) (R' = H or optionally substituted alkyl). Similarly, treatment of ester (**7** with *O*-alkylhydoxylamines or hydrazines gives the hydroxamate (R' = Oalkyl) or the hydrazide (R' = NH₂) respectively.

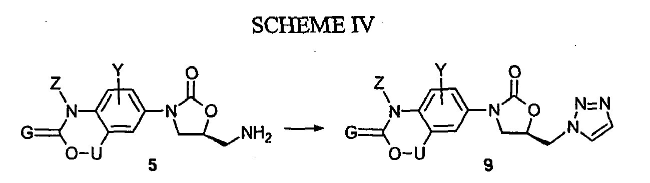

Triazole 1,3-benzoxazinone or 1,3-benzoxazepinone oxazolidinones **(9)** are most conveniently prepared, as shown in Scheme V, by reacting amine **(5)** with an arenesulfonyl hydrazone according to the methods of Ichikawa (Chem. Pharm. Bull., 2000, 48, 1947-1953) and Sakai (Bull. Chem. Soc. Jpn., 1986, 59, 179-184).

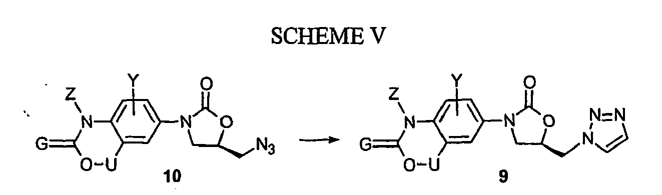

Alternatively, 1,3-benzoxazinone or 1,3-benzoxazepinone carbamate **(2)** is converted to the azido oxazolidinone **(10)** following the sequence of chemical transformations described by Brickner (J. Med. Chem., 1996, 39, 673-679). Cycloaddition of the intermediate azido compound with norbornadiene in a suitable solvent, such as dioxane at reaction temperatures in the range of about 50 °C to about 100°C affords the 1,2,3-triazolyl derivative (R' = H). Alternatively, a variety of other substituted triazoles (R' = Me, Cl, F, -OH, -CH₂OH,-CH₂CN, -CN, -C≡CH, -NH₂) may be prepared via cycloaddition in the presence of Cu(I) catalysis as described by Rostovtsec (Angew. Chem. Int. Ed., 2002, 41, 2596-2599) and subsequent chemical group modification by known methods when necessary.

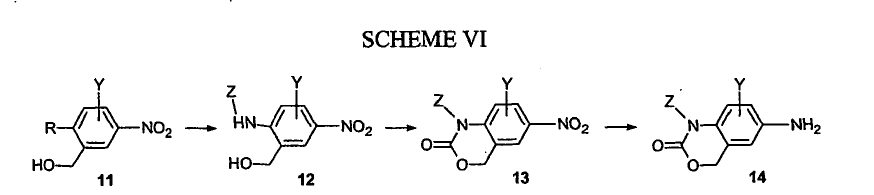

The requisite 1,3-benzoxazinone precursors may be prepared via nucleophilic aromatic substitution of a nitrobenzyl alcohol **(11)** bearing a leaving group R such as halogen, mesyloxy, tosyloxy or trifluoromethanesulfonyloxy with an amine (Z-NH₂) in a polar solvent such as dimethylsulfoxide or dimethylformamide at elevated temperature in the range of 60 to 120 °C to provide **(12).** The aminobenzyl alcohol **(12)** may be ring closed using a variety of methods known to one skilled in the art. For instance, treatment of structures **(12)** with 1,1'-carbonyldiimidazole in a solvent such as acetonitrile or tetrahydrofuran at an appropriate temperature, typically in a range of 20 °C to 60 °C, or with phosgene in a solvent such as toluene or methylene chloride, or mixtures thereof, in the presence of a base such as triethylamine at an appropriate temperature, typically in a range from -10 °C to 25 °C, affords the 1,3-benzoxazinone **(13).** Alternatively **(12)** may be reacted with an alkyl haloformate such as ethyl chloroformate in the presence of a base such as triethylamine or pyridine and then cyclized with excess metal alkoxide such as sodium methoxide in methanol to provide **(13).** The amino 1,3-benzoxazinone **(14)** is obtained from **(12)** via dissolving metal reduction or hydrogenation in the presence of a metal catalyst. 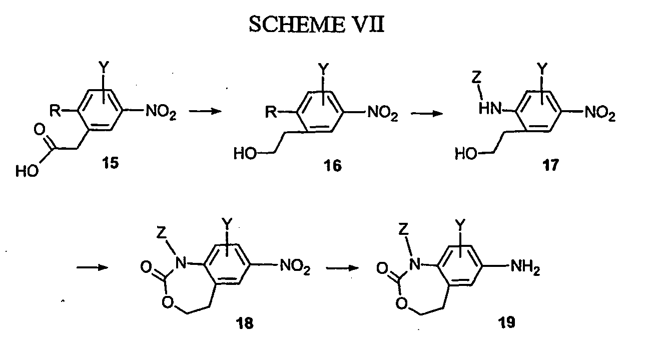 The requisite 1,3-benzoxazepinone precursors may be readily prepared via nucleophilic aromatic substitution of a nitrophenethyl alcohol **(16)** and following the procedure described in Scheme VI.

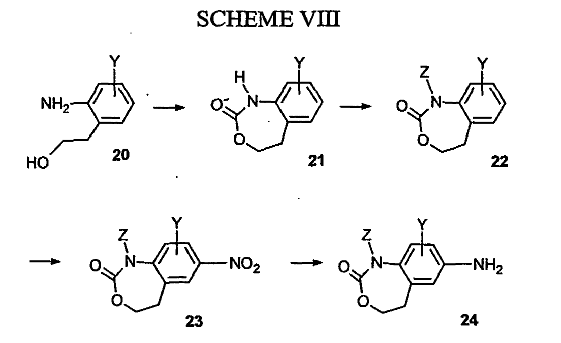

Alternatively, appropriately substituted aminophenethyl alcohols **(20)** may be may be ring closed using a variety of methods known to one skilled in the art. For instance, treatment of structures **(20)** with 1,1'-carbonyldiimidazole in a solvent such as acetonitrile or tetrahydrofuran at an appropriate temperature, typically in a range of 20 °C to 60 °C, or with phosgene in a solvent such as toluene or methylene chloride, or mixtures thereof, in the presence of a base such as triethylamine at an appropriate temperature, typically in a range from -10 °C to 25 °C, affords **(21).** The 1,3-benzoxazepinone **(21)** is alkylated with an alkyl halide, sulfate or tosylate in the presence of an organic or inorganic base such as 1,8-diazabicyclo[5.4.0]undec-7-ene, potassium carbonate or sodium hydride in a suitable solvent such as dimethylformamide at temperatures in the range 25 °C to 90 °C to provide **(22).** The amino 1,3-benzoxazepinone (24) is obtained from **(23)** via dissolving metal reduction or hydrogenation in the presence of a metal catalyst.

### Medical and Veterinary Uses

The compound of the present invention may be used for the treatment of infectious, Gram-positive bacterial infections caused by a variety of bacterial organisms, including those that require long-term therapy (>28 days).

Examples of the bacterial organisms include gram-positive bacteria such as multiple resistant staphylococci, for example *S. aureus and S. epidermidis*; multiple resistant streptococci, for example *S. pneumoniae* and *S. pyogenes*; and multiple resistant Enterococci, for example *E. faecalis*; gram negative aerobic bacteria such as Haemophilus, for example *H. influenzae* and Moraxella, for example *M. catarrhalis*; as well as anaerobic organisms such as bacteroides and clostridia species, and acid-fast organisms such as Mycobacteria, for example *M. tuberculosis*; and/or *Mycobacterium avium*. Other examples include Escherichia, for example *E. coli*. intercellular microbes, for example Chlamydia and Rickettsiae.

Examples of infections that may be treated with the compound of the present invention include central nervous system infections, external ear infections, infections of the middle ear, such as acute otitis media, infections of the cranial sinuses, eye infections, infections of the oral cavity, such as infections of the teeth, gums and mucosa, upper respiratory tract infections, lower respiratory tract infections, genitourinary infections, gastrointestinal infections, gynecological infections, septicemia, bone and joint infections, skin and skin structure infections, bacterial endocarditis, burns, antibacterial prophylaxis of surgery, and antibacterial prophylaxis in immunosuppressed patients, such as patients receiving cancer chemotherapy, or organ transplant patients. Specifically, infectious diseases that may be treated with the compound of the present invention are gram-positive infections such as osteomyelitis, endocarditis and diabetic foot.

### Antibacterial activity

The *in vitro* antibacterial activity of the compounds of the present invention may be assessed by following procedures recommended in (1) National Committee for Clinical Laboratory Standards (Jan. 2003), Methods for dilution antimicrobial tests for bacteria that grow aerobically, Approved Standard (6th ed), M7-A6, NCCLS, Wayne, PA; (2) National Committee for Clinical Laboratory Standards (Mar. 2001), Methods for antimicrobial susceptibility testing of anaerobic bacteria, Approved Standard (5th ed), M11-A4, NCCLS, Wayne, PA; (3) National Committee for Clinical Laboratory Standards (Jan.2003), MIC testing supplemental tables, M100-S13 (for use with M7-A6), NCCLS, Wayne, PA; and (4) Murray PR, Baron EJ, Jorgensen JH, et al. Manual of Clinical Microbiology (8th ed) Washington, DC: American Society for Microbiology Press, 2003. The antibacterial activity can be presented in the form of MIC value. The MIC value is the lowest concentration of drug, which prevented macroscopically visible growth under the conditions of the test. Table 1 lists the *in vitro* antibacterial activity of the present invention.

**Table 1**

| Results of *in vitro* antibacterial activity MICₛ (µg/mL) | | | |
|---|---|---|---|
| Example No. | *S. aureus* UC-76 SA-1 | *S. pneumoniae* SV1 SP-3 | *E.faecalis* MGH-2 EF 1-1 |
| 1 | 4 | 2 | 4 |
| 2 | 8 | 4 | 8 |
| 3 | 4 | 4 | 4 |
| 4 | 4 | 4 | 16 |
| 5 | 4 | 4 | 16 |
| 6 | 8 | 2 | 8 |
| 7 | 16 | 16 | 32 |
| 8 | 8 | 16 | 8 |
| 9 | 8 | 8 | 8 |
| 10 | 2 | 2 | 4 |
| 11 | 4 | 4 | 4 |
| 12 | 4 | 4 | 8 |
| 13 | 16 | 8 | 16 |
| 14 | 16 | 8 | 16 |
| 15 | 8 | 4 | 8 |
| 16 | 16 | 8 | 16 |
| 17 | 16 | 8 | 16 |
| 18 | 16 | 32 | 32 |
| 19 | 16 | 32 | 32 |
| 20 | 8 | 4 | 8 |
| 21 | 8 | 8 | 8 |
| 22 | 8 | 8 | 8 |

### Pharmaceutical Salts

The compound of formula I may be used in its native form or as a salt. In cases where forming a stable nontoxic acid or base salt is desired, administration of the compound as a pharmaceutically acceptable salt may be appropriate. Examples of pharmaceutically acceptable salts of the present invention include inorganic salts such as hydrochloride, hydrobromide, sulfate, nitrate, bicarbonate, carbonate salts, and organic salts such as tosylate, methanesulfonate, acetate, citrate, malonate, tartarate, succinate, benzoate, ascorbate, etoglutarate, and glycerophosphate. Pharmaceutically acceptable salts may be obtained using standard procedures well known in the art, for example, reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

### Routes of Administration

The oxazolidinone antibacterial prodrugs of this invention have useful activity against a variety of organisms including, but not limiting to, *Staphylococcus aureus, Staphylococcus epidermidis, Enterococcus faecium, Streptococcus pneumoniae, Streptococcus pyogenes, Enterococcus faecalis, Moraxella catarrhalis* and *H. influenzae*. In therapeutic use for treating, or combating, bacterial infections in a mammal (i.e. human and animals) an oxazolidinone prodrug of the present invention or its pharmaceutical compositions can be administered orally, parenterally, topically, rectally, transmucosally, or intestinally.

Parenteral administrations include indirect injections to generate a systemic effect or direct injections to the afflicted area. Examples of parenteral administrations are subcutaneous, intravenous, intramuscular, intradermal, intrathecal, intraocular, intranasal, intravetricular injections or infusions techniques.

Topical administrations include the treatment of infectious areas or organs readily accessibly by local application, such as, for example, eyes, ears including external and middle ear infections, vaginal, open wound, skins including the surface skin and the underneath dermal structures, or other lower intestinal tract. It also includes transdermal delivery to generate a systemic effect.

The rectal administration includes the form of suppositories.

The transmucosal administration includes nasal aerosol or inhalation applications.

The preferred routes of administration are oral and parenteral.

### Composition/Formulation

Pharmaceutical compositions of the present invention may be manufactured by processes well known in the art, *e*.*g*., by means of conventional mixing, dissolving, granulation, dragee-making, levigating, emulsifying, encapsulating, entrapping, lyophilizing processes or spray drying.

Pharmaceutical compositions for use in accordance with the present invention may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For oral administration, the compounds can be formulated by combining the active compounds with pharmaceutically acceptable carriers well known in the art. Such carriers enable the compounds of the invention to be formulated as tablets, pills, lozenges, dragees, capsules, liquids, solutions, emulsions, gels, syrups, slurries, suspensions and the like, for oral ingestion by a patient. A carrier can be at least one substance which may also function as a diluent, flavoring agent, solubilizer, lubricant, suspending agent, binder, tablet disintegrating agent, and encapsulating agent. Examples of such carriers or excipients include, but are not limited to, magnesium carbonate, magnesium stearate, talc, sugar, lactose, sucrose, pectin, dextrin, mannitol, sorbitol, starches, gelatin, cellulosic materials, low melting wax, cocoa butter or powder, polymers such as polyethylene glycols and other pharmaceutical acceptable materials.

Dragee cores are provided with suitable coatings. For this purpose, concentrated sugar solutions may be used which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions which can be used orally include push-fit capsules made of gelatin, as well as soft, sealed capsules made of gelatin and a plasticizer, such as glycerol or sorbitol. The push-fit capsules can contain the active ingredients in admixture with a filler such as lactose, a binder such as starch, and/or a lubricant such as talc or magnesium stearate and, optionally, stabilizers. In soft capsules, the active compounds may be dissolved or suspended in suitable liquids, such as fatty oils, liquid paraffin, liquid polyethylene glycols, cremophor, capmul, medium or long chain mono-, di- or triglycerides. Stabilizers may be added in these formulations, also.

Liquid form compositions include solutions, suspensions and emulsions. For example, there may be provided solutions of the compounds of this invention dissolved in water and water-propylene glycol and water-polyethylene glycol systems, optionally containing suitable conventional coloring agents, flavoring agents, stabilizers and thickening agents.

The compounds may also be formulated for parenteral administration, *e*.*g*., by injections, bolus injection or continuous infusion. Formulations for parenteral administration may be presented in unit dosage form, *e*.*g*., in ampoules or in multi-dose containers, with an added preservative. The compositions may take such forms as suspensions, solutions or emulsions in oily or aqueous vehicles, and may contain formulating materials such as suspending, stabilizing and/or dispersing agents.

For injection, the compounds of the invention may be formulated in aqueous solution, preferably in physiologically compatible buffers or physiological saline buffer. Suitable buffering agents include trisodium orthophosphate, sodium bicarbonate, sodium citrate, N-methylglucamine, L(+)-lysine and L(+)-arginine.

Parenteral administrations also include aqueous solutions of a water soluble form, such as, without limitation, a salt, of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances which increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions.

Alternatively, the active ingredient may be in powder form for constitution with a suitable vehicle, *e*.*g*., sterile, pyrogen-free water, before use. For suppository administration, the compounds may also be formulated by mixing the agent with a suitable non-irritating excipient which is solid at room temperature but liquid at rectal temperature and therefore will melt in the rectum to release the drug. Such materials include cocoa butter, beeswax and other glycerides.

For administration by inhalation, compounds of the present invention can be conveniently delivered through an aerosol spray in the form of solution, dry powder, or suspensions. The aerosol may use a pressurized pack or a nebulizer and a suitable propellant. In the case of a pressurized aerosol, the dosage unit may be controlled by providing a valve to deliver a metered amount. Capsules and cartridges of, for example, gelatin for use in an inhaler may be formulated containing a power base such as lactose or starch.

For topical applications, the pharmaceutical composition may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion such as suspensions, emulsion, or cream containing the active components suspended or dissolved in one or more pharmaceutically acceptable carriers. Suitable carriers include, but are not limited to, mineral oil, sorbitan monostearate, polysorbate 60, cetyl esters wax, ceteary alcohol, 2-octyldodecanol, benzyl alcohol and water.

For ophthalmic and otitis uses, the pharmaceutical compositions may be formulated as micronized suspensions in isotonic, pH adjusted sterile saline, or preferably, as solutions in isotonic, pH adjusted sterile saline, either with or without a preservative such as a benzylalkonium chloride. Alternatively, for ophthalmic uses, the pharmaceutical compositions may be formulated in an ointment such as petrolatum.

In addition to the formulations described previously, the compounds may also be formulated as depot preparations. Such long acting formulations may be in the form of implants. A compound of this invention may be formulated for this route of administration with suitable polymers, hydrophobic materials, or as a sparing soluble derivative such as, without limitation, a sparingly soluble salt.

Additionally, the compounds may be delivered using a sustained-release system. Various sustained-release materials have been established and are well known by those skilled in the art. Sustained-release capsules may, depending on their chemical nature, release the compounds for 24 hours or for up to several days.

### Dosage

Pharmaceutical compositions suitable for use in the present invention include compositions wherein the active ingredients are contained in an amount sufficient to achieve the intended purpose, i.e., the treatment or prevent of infectious diseases. More specifically, a therapeutically effective amount means an amount of compound effective to prevent, alleviate or ameliorate symptoms of disease or prolong the survival of the subject being treated.

The quantity of active component, that is the compound of this invention, in the pharmaceutical composition and unit dosage form thereof may be varied or adjusted widely depending upon the manner of administration, the potency of the particular compound and the desired concentration. Determination of a therapeutically effective amount is well within the capability of those skilled in the art. Generally, the quantity of active component will range between 0.5% to 90% by weight of the composition.

Generally, a therapeutically effective amount of dosage of active component will be in the range of about 0.1 to about 400 mg/kg of body weight/day, more preferably about 1.0 to about 50 mg/kg of body weight/day. It is to be understood that the dosages may vary depending upon the requirements of each subject and the severity of the bacterial infection being treated. In average, the effective amount of active component is about 200 mg to 800 mg and preferable 600 mg per day.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

Also, it is to be understood that the initial dosage administered may be increased beyond the above upper level in order to rapidly achieve the desired plasma concentration. On the other hand, the initial dosage may be smaller than the optimum and the daily dosage may be progressively increased during the course of treatment depending on the particular situation. If desired, the daily dose may also be divided into multiple doses for administration, e.g., two to four times per day.

In cases of local administration or selective uptake, the effective local concentration of the drug may not be related to plasma concentration and other procedures know in the art may be used to determine the desired dosage amount.

### EXAMPLES

In the discussion above and in the examples below, the following abbreviations have the following meanings. If an abbreviation is not defined, it has its generally accepted meaning.
- bm =: broad multiplet
- BOC =: *tert*-butoxycarbonyl
- bd =: broad doublet
- bs =: broad singlet
- CDI =: 1,1*O*-carbodiimidazole
- d =: doublet
- dd =: doublet of doublets
- dq =: doublet of quartets
- dt =: doublet of triplets
- DMF =: dimethylformamide
- DMAP =: dimethylaminopyridine
- DMSO =: dimethyl sulfoxide
- eq. =: equivalents
- g =: grams
- h =: hours
- HPLC =: high pressure liquid chromatography
- HATU =: N-[(dimethylamino)-1H-1,2,3-triazolo-[4,5-b]pyridin-1-yl-methylene]-N-methylmethanaminium hexafluorophosphate N-oxide
- LG =: leaving group
- m =: multiplet
- M =: molar
- M% =: mole percent
- max =: maximum
- meq =: milliequivalent
- mg =: milligram
- mL =: milliliter
- mm =: millimeter
- mmol =: millimol
- q =: quartet
- s =: singlet
- t or tr =: triplet
- TBS =: tributylsilyl
- TFA =: trifluoroacetic acid
- THF =: tetrahydrofuran
- TLC =: thin layer chromatography
- p-TLC =: preparative thin layer chromatography
- µL =: microliter
- N =: normality
- MeOH =: methanol
- DCM =: dichloromethane
- HCl =: hydrochloric acid
- ACN =: acetonitrile
- MS =: mass spectrometry
- rt =: room temperature
- EtOAc =: ethyl acetate
- EtO =: ethoxy
- Ac =: acetate
- NMP =: 1-methyl-2-pyrrolidinone
- µL =: microliter
- J =: coupling constant
- NMR =: Nuclear magnetic resonance
- MHz =: megahertz
- Hz =: hertz
- m/z =: mass to charge ratio
- min =: minutes
- Boc =: tert-butoxycarbonyl
- CBZ =: benzyloxycarbonyl
- DCC =: 1,3-dicyclohexylcarbodiimide
- PyBop =: benzotriazole-1-yl-oxy-trispyrrolidinophosphonium hexafluorophosphate

### Example 1 Preparation of (S)-N-((3-(1-methyl-2-oxo-2,4-dihydro-1H-benzo[d][1,3]oxazin-6-yl)-2-oxooxazolidin-5-yl)methyl)acetamide

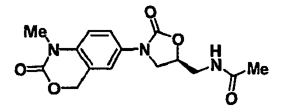

### Step 1: Preparation of (2-(methylamino)-5-nitrophenyl)methanol.

Methylamine (40% aqueous solution, 3.05 mL, 0.040 mol) is added to (2-fluoro-5-nitrophenyl)methanol (2.0 g, 0.013 mol) in dimethylsulfoxide (20 mL) and the mixture heated at 45 °C for 30 minutes. The reaction is diluted with water and extracted with dichloromethane. The organic layer is separated, washed with water, citric acid and brine, dried (Na₂SO₄), and evaporated to give the title compound. HPLC (SYMMETRY C₁₈ 3.5 µM, 4.6 x 30 mm column; gradient elution 2%-98% MeCN with 0.1 % TFA over 10 min; 2 mL/min rate): retention time = 3.97; MS (m/z): (M-H)⁻181.0.

### Step 2: Preparation of 1-methyl-6-nitro-1H-benzo[d][1,3]oxazin-2(4H)-one.

1,1'-Carbonyldiimidazole (1.92 g, 0.012 mol) is added to (2-(methylamino)-5-nitrophenyl)methanol (1.89 g, 0.012 mol) in acetonitrile (20 mL) and the mixture heated at 60 °C for 6 hours. The reaction mixture is diluted with water and extracted with dichloromethane. The organic layer is separated, washed with water and brine, dried (Na₂SO₄), and evaporated. The residue is purified by flash column chromatography to give the title compound. HPLC (SYMMETRY C₁₈ 3.5 µM, 4.6 x 30 mm column; gradient elution 2%-98% MeCN with 0.1 % TFA over 10 min; 2 mL/min rate): retention time = 4.09 min; MS (m/z): (M-H)⁻ 207.0.

### Step 3. Preparation of 6-amino-1-methyl-1H-benzo[d][1,3]oxazin-2(4H)-one.

Iron powder (2.32 g, 0.042 mol) is added portionwise to 1-methyl-6-nitro-1H-benzo[d][1,3]oxazin-2(4H)-one (2.2 g, 0.011 mol) and ammonium chloride (5.62 g, 0.106 mol) in ethanol (40 mL) and water (20 mL) at 90 °C. The mixture is stirred vigorously and heated for 1 hour, allowed to cool to room temperature, and diluted with dichloromethane (500 mL). The mixture is filtered through celite, washed with water and brine, dried (Na₂SO₄), and evaporated to give the title compound as a brown solid. HPLC (SYMMETRY C₁₈ 3.5 µM, 4.6 x 30 mm column; gradient elution 2%-98% MeCN with 0.1% TFA over 10 min; 2 mL/min rate): retention time = 1.258 min; MS (m/z): (M+H)⁺ 178.9. Step 4. Preparation of benzyl 1-methyl-2-oxo-2,4-dihydro-1H-benzo[d][1,3]oxazin-6-ylcarbamate.

Benzyl chloroformate (1.7 mL, 0.011 mol) is added dropwise to 6-amino-1-methyl-1H-benzo[d][1,3]oxazin-2(4H)-one (2.0 g, 0.011 mol) and pyridine (1.33 mL, 0.017 mol) in dichloromethane (25 mL) at 0 °C. The mixture is stirred at 0 °C for 30 min, allowed to warm to room temperature and then diluted with water. The organic layer is separated, washed with brine, dried (Na₂SO₄), and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 5.07 min; MS (m/z): (M+H)⁺ 313.1.

### Step 5: Preparation of (S)-tert-butyl (3-(1-methyl-2-oxo-2,4-dihydro-1H-benzo[d][1,3]oxazin-6-yl)-2-oxooxazolidin-5-yl)methylcarbamate.

Lithium *t*-butoxide (1.0 M solution in THF, 13.53 mL, 0.014 mol) is added dropwise to benzyl 1-methyl-2-oxo-2,4-dihydro-1H-benzo[d][1,3]oxazin-6-ylcarbamate (1.76 g, 0.006 mol) and (*S*)-*tert*-butyl 3-chloro-2-hydroxypropylcarbamate (1.42 g, 0.007 mol) in DMF (10 mL) at 0°C. The mixture is allowed to warm to room temperature and stirred for 14 hours. The reaction is quenched with saturated aqueous ammonium chloride, diluted with water and extracted with dichloromethane. The organic layer is washed with brine, dried (Na₂SO₄), and evaporated. The residue is purified by flash column chromatography (20% EtOAc/Hexane) to give the title compound. (SYMMETRY C₁₈ 3.5 µM, 4.6 x 30 mm column; gradient elution 2%-98% MeCN with 0.1% TFA over 10 min; 2 mL/min rate): retention time = 4.45 min; MS (m/z): (M+H)⁺ 378.3.

### Step 6: Preparation of (S)-6-(5-(aminomethyl)-2-oxooxazolidin-3-yl)-1-methyl-1H-benzo[d][1,3]oxazin-2(4H)-one.

(*S*)-*tert*-Butyl (3-(1-methyl-2-oxo-2,4-dihydro-1H-benzo[d][1,3]oxazin-6-yl)-2-oxooxazolidin-5-yl)methylcarbamate (1.5 g, 0.004 mol) and 50% trifluoroacetic acid/dichloromethane (5 mL) are stirred at room temperature for 1 hour. The mixture is evaporated to give the title compound as the TFA salt. HPLC (SYMMETRY C₁₈ 3.5 µM, 4.6 x 30 mm column; gradient elution 2%-98% MeCN with 0.1% TFA over 10 min; 2 mL/min rate): retention time = 2.621 min; MS (m/z): (M+H)⁺ 278.3.

### Step 7: Preparation of (S)-N-((3-(1-methyl-2-oxo-2,4-dihydro-1H-benzo[d][1,3]oxazin-6-yl)-2-oxooxazolidin-5-yl)methyl)acetamide.

Acetic anhydride (0.187 mL, 0.0012 mol) is added dropwise to (S)-6-(5-(aminomethyl)-2-oxooxazolidin-3-yl)-1-methyl-1H-benzo[d][1,3]oxazin-2(4H)-one (0.42 g, 0.0015 mol) and pyridine (0.36 mL, 0.0045 mol) in dichloromethane (5 mL) at 0°C. The mixture is stirred at 0 °C for 30 minutes and then allowed to warm to room temperature. The reaction is diluted with dichloromethane, washed with water, citric acid and brine, dried (Na₂SO₄) and evaporated. The residue is purified by preparative TLC (10% MeOH/DCM) to give the title compound. (SYMMETRY C₁₈ 3.5 µM, 4.6 x 30 mm column; gradient elution 2%-98% MeCN with 0.1% TFA over 10 min; 2 mL/min rate): retention time = 3.172 min; ¹H NMR (300 MHz, DMSO-*d₆*) 7.49 (d, *J* = 5.8 Hz, 2H), 7.11(d, *J* = 9.6 Hz, 1H), 5.24 (s, 2H), 4.71 (m, 1H), 4.09 (t, *J* = 8.3 Hz, 1H), 3.71 (dd, *J* = 8.0, 2.9 Hz, 1H), 3.41 (dd, *J* = 5.5, 5.1 Hz, 2H), 3.25 (s, 3H), 1.82 (s, 3H); MS for C₁₅H₁₇N₃O₅ m/z 320.1(M+H)⁺.

### Example 2 Preparation of (S)-N-((3-(1-methyl-2-oxo-2,4-dihydro-1H-benzo[d][1,3]oxazin-6-yl)-2-oxooxazolidin-5-yl)methyl)propionamide

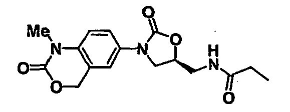

Propionic anhydride (0.23 mL, 0.0018 mol) is added dropwise to (S)-6-(5-(aminomethyl)-2-oxooxazolidm-3-yl)-1-methyl-1H-benzo[d][1,3]oxazin-2(4H)-one (0.50 g, 0.0018 mol) and pyridine (0.44 mL, 0.0054 mol) in dichloromethane (5 mL) at 0 °C. The mixture is stirred at 0 °C for 30 minutes and then allowed to warm to room temperature. The reaction is diluted with dichloromethane, washed with water, citric acid and brine, dried (Na₂SO₄) and evaporated. The residue is purified by preparative TLC (10% MeOH/DCM) to give the title compound. HPLC (SYMMETRY C₁₈ 3.5 µM, 4.6 x 30 mm column; gradient elution 2%-98% MeCN with 0.1 % TFA over 10 min; 2 mL/min rate): retention time = 3.53 min. ¹H NMR (300 MHz, DMSO-d₆) δ 8.16 (br s, 1H), 7.47 (d, J = 9 Hz, 2H), 7.10 (d, J = 9 Hz, 2H), 5.24 (s, 2H), 4.71-4.69 (m, 1H), 4.08 (t, J = 9 Hz, 1H), 3.74-3.69 (m, 1H), 3.43-3.25 (m, 5H), 2.10 (q, J = 9 Hz, 2H), 0.94-0.85 (m, 3H); MS for C₁₆H₁₉N₃O₅ m/z 334.2 (M+H)⁺.

### Example 3 Preparation of (S)-methyl (3-(1-methyl-2-oxo-2,4-dihydro-1H-benzo[d][1,3]oxazin-6-yl)-2-oxooxazolidin-5-yl)methylcarbamate

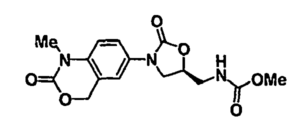

Methyl chloroformate (0.196 mL, 0.0021 mol) is added dropwise to (*S*)-6-(5-(aminomethyl)-2-oxooxazolidin-3-yl)-1-methyl-1H-benzo[d][1,3]oxazin-2(4H)-one (0.49 g, 0.0018 mol) and N,N-diisopropylethylamine (0.685 mL, 0.0053 mol) in dichloromethane (5 mL) at 0 °C. The mixture is stirred at 0 °C for 30 minutes and then allowed to warm to room temperature. The reaction is diluted with dichloromethane, washed with water, citric acid and brine, dried (Na₂SO₄), and evaporated. The residue is purified by preparative TLC (10% MeOH/DCM) to give the title compound. HPLC (SYMMETRY C₁₈ 3.5 µM, 4.6 x 30 mm column; gradient elution 2%-98% MeCN with 0.1 % TFA over 10 min; 2 mL/min rate): retention time = 3.486 min; ¹H NMR (300 MHz, DMSO-*d₆*) 7.47 (d, *J* = 9.9 Hz, 2H), 7.11 (d, *J* = 8.2 Hz, 1H), 5.24 (s, 2H), 4.71 (m, 1H), 4.09 (t, *J* = 8.8 Hz, 1H), 3.75 (dd, *J* = 6.3, 2.9 Hz, 1H), 3.53 (s, 3H), 3.33 (t, *J* = 5.5 Hz, 2H), 3.25 (s, 3H); MS for C₁₅H₁₇N₃O₆ m/z 336.1(M+H)⁺.

### Example 4 Preparation of (R)-3-(1-methyl-2-oxo-2,4-dihydro-1H-benzo[d][1,3]oxazin-6-yl)-2-oxooxazolidine-5-carboxamide

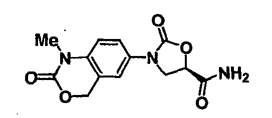

### Step 1: Preparation of 6-nitro-1H-benzo[d][1,3]oxazin-2(4H)-one.

1,1'-Carbonyldiimidazole (2.78 g, 17.02 mmol) is added in one portion to a suspension of (2-amino-5-nitro-phenyl)-methanol (2.60 g, 15.4 mmol) in dioxane (20 mL) and the mixture stirred overnight at room temperature. The reaction is quenched with 0.1 N HCl (25 mL) followed by extraction with ethyl acetate (50 mL X 3). The combined organic phases are washed with water (50 mL), sat. Na₂CO₃ (50 mL), brine (50 mL), dried (Na₂SO₄) and concentrated under reduced pressure. The residue is purified by flash column chromatography (ethyl acetate) to obtain the title compound.

### Step 2: Preparation of 1-methyl-6-nitro-1H-benzo[d][1,3]oxazin-2(4H)-one.

Methyl iodide (0.57 mL. 9.27 mmol) is added to 6-nitro-1H-benzo[d][1,3]oxazin-2(4H)-one (0.90 g, 4.63 mmol) and potassium carbonate (1.60 g, 11.57 mmol) in DMF (20 mL). The mixture is stirred for 16 hours and diluted with water. The resulting precipitate is filtered and dried to obtain the title compound.

### Step 3: Preparation of 6-amino-1-methyl-1H-benzo[d][1,3]oxazin-2(4H)-one.

Iron (0.6 g, 10.80 mmol) is added portionwise to a vigorously stirred solution of 1-methyl-6-nitro-1H-benzo[d][1,3]oxazin-2(4H)-one (750 mg, 3.60 mmol) and ammonium chloride (1.92 g, 36.00 mmol) in ethanol (20 mL) and water (10 mL) at 80 °C. The mixture is stirred and heated for 2 h, diluted with dichloromethane (200 mL) and filtered. The organic layer is separated, washed with brine, dried (Na₂SO₄) and evaporated to give the title compound.

### Step 4: Preparation of (R)-methyl 2-hydroxy-3-(1-methyl-2-oxo-2,4-dihydro-1H-benzo[d][1,3]oxazin-6-ylamino)propanoate.

6-Amino-1-methyl-1H-benzo[d][1,3]oxazin-2(4H)-one (580 mg, 3.53 mmol), lithium triflate (605 mg, 3.88 mmol), and methyl-(*R*)-glycidate (396 mg, 3.88 mmol) in acetonitrile (5 mL) are heated at 50 °C overnight. The mixture is diluted with water and extracted with ethyl acetate. The extract is washed with brine, dried (Na₂SO₄) and evaporated. The residue is purified by filtration through a short silica gel column eluting with ethyl acetate and used directly in the next step.

### Step 5: Preparation of (R)-methyl 3-(1-methyl-2-oxo-2,4-dihydro-1H-benzo[d][1,3]oxazin-6-yl)-2-oxooxazolidine-5-carboxylate.

(*R*)-Methyl2-hydroxy-3-(1-methyl-2-oxo-2,4-dihydro-1H-benzo[d][1,3]oxazin-6-ylamino)propanoate (from above, Step 4) and 1,1'-carbonyldiimidazole (1.14 g, 7.06 mmol) in acetonitrile (20 mL) are stirred overnight at room temperature. The mixture is diluted with 0.1 N HCl (10 mL). and the resulting pale yellow precipitate filtered, washed with water, and dried under vacuum to give (5*R*)-3-(1-Methyl-2-oxo-1,4-dihydro-2*H*-benzo[*d*][1,3]oxazin-6-yl)-2-oxo-oxazolidine-5-carboxylic acid methyl ester.

### Step 6: Preparation of (R)-3-(1-methyl-2-oxo-2,4-dihydro-1H-benzo[d][1,3]oxazin-6-yl)-2-oxooxazolidine-5-carboxamide.

Ammonia (2M solution in methanol, 2.5 mL, 5 mmol) and (*R*)-methyl 3-(1-methyl-2-oxo-2,4-dihydro-1H-benzo[d][1,3]oxazin-6-yl)-2-oxooxazolidine-5-carboxylate (50 mg, 0.16 mmol) are stirred overnight at room temperature. The mixture is evaporated to dryness, the resulting solid washed with cold methanol, and dried under vacuum to give the title compound. ¹H NMR (300 MHz, DMSO,) δ 7.87 (br s, 1H), 7.60 (br s, 1H), 7.54-7.52 (m, 2H), 7.12-7.09 (m, 1H), 5.24 (s, 2H), 5.01 (dd, *J* = 6.0 Hz, 9.6 Hz, 1H), 4.25 (t, *J* = 9.3 Hz, 1H), 3.98 (dd, *J* = 6.0 Hz, 8.8 Hz, 1H), 3.25 (s, 3H); MS for C₁₃H₁₃N₃O₅ m/z 292.0(M+H)⁺

### Example 5 Preparation of (R)-N-methyl-3-(1-methyl-2-oxo-2,4-dihydro-1H-benzo[d][1,3]oxazin-6-yl)-2-oxooxazolidine-5-carboxamide

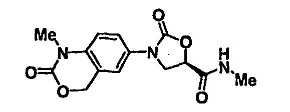

Methylamine (2M solution in methanol, 2.5 mL, 5 mmol) and (*R*)-methyl 3-(1-methyl-2-oxo-2,4-dihydro-1H-benzo[d][1,3]oxazin-6-yl)-2-oxooxazolidine-5-carboxylate (see Example 4, Step 6) are stirred overnight at room temperature. The mixture is evaporated to dryness, the resulting solid washed with cold methanol, and dried under vacuum to give the title compound. ¹H NMR (300 MHz, DMSO,) δ 8.37 (br d, *J* = 4.94 Hz, 1H), 7.53-7.51 (m, 2H), 7.12-7.08 (m, 1H), 5.23 (s, 2H), 5.05 (dd, *J* = 6.04 Hz, 9.61 Hz, 1H), 4.25 (t, *J* = 9.61 Hz,, 1H), 3.99 (dd, *J* = 6.04 Hz, 8.79 Hz 1H), 3.25 (s, 3H), 2.64 (d, *J* = 4.39 Hz, 2H); MS for C₁₄H₁₅N₃O₅ m/z 306.5(M+H)⁺.

### Example 6 Preparation of (S)-Fluoromethyl (3-(1-methyl-2-oxo-2,4-dihydro-1H-benzo[d][1,3]oxazin-6-yl)-2-oxooxazolidin-5-yl)methylcarbamate

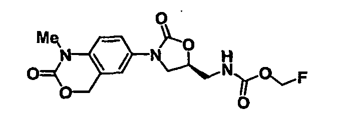

Chloromethyl chloroformate (0.058 g, 0.0005 mol) is added dropwise to potassium fluoride (0.168 g, 0.003 mol) and 18-crown-6 (0.057 g, 0.0002 mol) in acetonitrile (4 mL) and stirred at room temperature for 12 h. The reaction is cooled to 0 °C and a mixture of (*S*)-6-(5-(aminomethyl)-2-oxooxazolidin-3-yl)-1-methyl-1*H*-benzo[*d*][1,3]oxazin-2(4H)-one (0.2 g, 0.0007 mol) and triethylamine (0.15 mL, 0.0015 mol) in acetonitrile (2 mL) is added dropwise. The reaction mixture is stirred for 30 min at 0 °C and then allowed to warm to room temperature. The reaction mixture is diluted with ether, washed with water, citric acid and brine, dried (Na₂SO₄) and evaporated. The residue is purified by preparative TLC (10% MeOH/DCM) to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.623 min; ¹H NMR (300 MHz, DMSO-*d₆*) 7.48 (d, *J* = 5.8 Hz, 2H), 7.12 (d, *J* = 9.6 Hz, 1H), 5.66(d, *J* = 53.3 Hz, 2H), 5.24 (s, 2H), 4.71 (m, 1H), 4.09 (t, *J* = 8.3 Hz, 1H), 3.72 (dd, *J* = 8.0, 2.9 Hz, 1H), 3.40 (q, *J* = 5.5, 5.1 Hz, 2H), 3.24 (s, 3H); MS for C₁₅H₁₆N₃O₆ m/z 353.9 (M+H)⁺.

### Example 7 Preparation of (R)-6-(5-((1H-1,2,3-triazol-1-yl)methyl)-2-oxooxazolidin-3-yl)-1-methyl-1H-benzo[d][1,3]oxazin-2(4H)-one

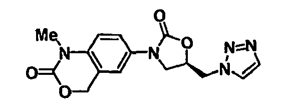

### Step 1: Preparation of (R)-6-(5-(hydroxymethyl)-2-oxooxazolidin-3-yl)-1-methyl-1H-benzo[d][1,3]oxazin-2(4H)-one.

Lithium bis(trimethylsilyl)amide in tetrahydrofuran (1.0M solution in THF, 19.2 mL, 0.0192 mol) is added to benzyl 1-methyl-2-oxo-2,4-dihydro-1H-benzo[d][1,3]oxazin-6-ylcarbamate (3.00 g, 0.0096 mol) in tetrahydrofuran (35 mL) at -78 °C and stirred for 30 minutes. (R)-(-)-Glycidyl butyrate (1.50 mL, 0.0106 mol) is added and the mixture allowed to warm to room temperature and stirred overnight. The reaction is diluted with dichloromethane, washed with saturated ammonium chloride solution and brine, dried (MgSO₄), and concentrated. The residue is purified by column chromatography (20% Hexanes/EtOAc) to afford the title compound. MS for C₁₃H₁₄N₂O₅ m/z 279.3(M+H)⁺. Step 2: Preparation of (R)-(3-(1-methyl-2-oxo-2,4-dihydro-1H-benzo[d][1,3]oxazin-6-yl)-2-oxooxazolidin-5-yl)methyl methanesulfonate.

Methanesulfonyl chloride (0.77 mL, 0.010 mol) is added dropwise at 0 °C to (R)-6-(5-(hydroxymethyl)-2-oxooxazolidin-3-yl)-1-methyl-1H-benzo[d][1,3]oxazin-2(4H)-one (2.9 g, 0.010 mol) and triethylamine (2.09 ml, 0.015 mol) in dichloromethane ( 25 ml) and the mixture stirred for 45 minutes. The reaction is quenched with saturated sodium bicarbonate, diluted with water and extracted with dichloromethane. The organic layer is washed with brine, dried (Na₂SO₄) and evaporated to give product used directly in the next step without purification; MS for C₁₄H₁₆N₂O₇S m/z 357.3(M+H)⁺.

### Step 3: Preparation of (R)-6-(5-(azidomethyl)-2-oxooxazolidin-3-yl)-1-methyl-1H-benzo[d][1,3]oxazin-2(4H)-one.

(R)-(3-(1-methyl-2-oxo-2,4-dihydro-1H-benzo[d][1,3]oxazin-6-yl)-2-oxooxazolidin-5-yl)methyl methanesulfonate (3.2 g, 0.0090 mol) and sodium azide (2.92 g, 0.045 mol) in dimethylformamide (15 ml) is heated at 70 °C for 16 h. The reaction is diluted with water and extracted with dichloromethane. The organic layer is washed with brine, dried (Na₂SO₄) and evaporated. The residue is purified by flash column chromatography (20% EtOAc/Hexane) to give the title compound. MS for C₁₃H₁₃N₅O₄ m/z 304.3.3 (M+H)⁺.

### Step 4: Preparation of (R)-6-(5-((1H-1,2,3-triazol-1-yl)methyl)-2-oxooxazolidin-3-yl)-1-methyl-1H-benzo[d][1,3]oxazin-2(4H)-one.

Bicyclo[2.2.1]hepta-2,5-diene (0.43 ml, 0.0040 mol) and (R)-6-(5-(azidomethyl)-2-oxooxazolidin-3-yl)-1-methyl-1H-benzo[d][1,3]oxazin-2(4H)-one (0.6 g, 0.0020 mol) in dioxane (20 ml) are heated at 70°C for 14 h. The solvent is removed under reduced pressure and the residue purified by flash column chromatography (20% EtOAc/Hexane) to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.48 min; ¹H NMR (300 MHz, DMSO-d₆) δ 7.77-7.72 (m, 1H), 7.32-7.16 (m, 2H), 6.88-6.85 (m, 1H), 5.15-5.03 (m, 3H), 4.78 (d, J = 6 Hz, 2H), 4.16-4.10 (m, 1H), 3.98-3.93-1H), 3.34 (s, 3H); MS for C₁₅H₁₅N₅O₄ m/z 330.1 (M+H)⁺.

### Example 8 Preparation of (R)-3-(8-fluoro-1-methyl-2-oxo-2,4-dihydro-1H-benzo[d][1,3]oxazin-6-yl)-2-oxooxazolidine-5-carboxamide

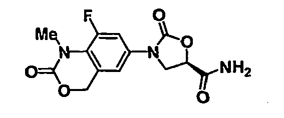

### Step 1: Preparation of (2,3-difluorobenzyl)acetate.

Acetic anhydride (7.29mL, 0.071 mol) is added at 0 °C to 2,3-difluorobenzyl alcohol (8.5 g, 0.059 mol) and diisopropylethylamine (20.5 mL, 0.118 mol) in dichloromethane (100 mL), stirred for 30 min. at 0 °C, and then allowed to warm to room temperature. The reaction is diluted with dichloromethane, the organic layer separated, washed with water, citric acid and brine, dried (Na₂SO₄) and evaporated to afford the title compound. HPLC (SYMMETRY C₁₈ 3.5 µM, 4.6 x 30 mm column; gradient elution 2%-98% MeCN with 0.1% TFA over 10 min; 2 mL/min rate): retention time = 5.152 min; MS (m/z): (M+H)⁺ 187.2

### Step 2: Preparation of 2,3-difluoro-5-nitrobenzyl acetate.

2,3-Difluorobenzyl acetate (8 g, 0.043 mol) is added dropwise at -10 °C to fuming nitric acid (40 mL) and stirred at that temperature for 1 hour. The reaction is diluted with ice water and extracted with dichloromethane. The organic layer is separated, washed with water, citric acid and brine, dried (Na₂SO₄) and evaporated to give a mixture of the desired 5-nitro (40%) and 4-nitro (60%) regioisomers. The mixture is used directly in the next step. HPLC (SYMMETRY C₁₈ 3.5 µM, 4.6 x 30 mm column; gradient elution 2%-98% MeCN with 0.1 % TFA over 10 min; 2 mL/min rate): retention time = 5.135 and 5.353 min; MS (m/z): (M-H)⁺ 230.0.

### Step 3: Preparation of (2,3-difluoro-5-nitrophenyl)methanol.

Potassium carbonate (3.584g, 0.026 mol) and 2,3-difluoro-5-nitrobenzyl acetate (mixture, Step 2, 3.0 g, 0.013mol) in 6:1 methanol- water (30 mL) are stirred at room temperature for 1h. Methanol is removed under reduced pressure and the remaining aqueous solution acidified with 2N HCl and extracted with ethyl acetate. The extract is washed with water and brine, dried (Na₂SO₄) and evaporated. The resulting residue is purified by flash column chromatography to give the title compound as a mixture of 4- and 5-nitro regioisomers. HPLC (SYMMETRY C₁₈ 3.5 µM, 4.6 x 30 mm column; gradient elution 2%-98% MeCN with 0.1% TFA over 10 min; 2 mL/min rate): retention time = 3.733 min; MS (m/z): (M-H)⁺ 188.1.

### Step 4: Preparation of (3-fluoro-2-(methylamino)-5-nitrophenyl)methanol.

Methylamine (40% by weight in water, 1.2 mL, 0.017 mol) and (2,3-difluoro-5-nitrophenyl)methanol (1.1 g, 0.0058 mol) in dimethylsulfoxide (20 mL) are heated at 100 °C for 30 minutes. The reaction mixture is diluted with ice water and extracted with dichloromethane. The organic layer is separated, washed with water, citric acid and brine, dried (Na₂SO₄) and evaporated to give mixture of 4- and 5-nitro regioisomers suitable for use directly in the next step. HPLC (SYMMETRY C₁₈ 3.5 µM, 4.6 x 30 mm column; gradient elution 2%-98% MeCN with 0.1% TFA over 10 min; 2 mL/min rate): retention time = 4.49 and 4.23 min; MS (m/z): (M-H)⁺ 199.0.

### Step 5: Preparation of 8-fluoro-1-methyl-6-nitro-1H-benzo[d][1,3]oxazin-2(4H)-one.

1,1'-Carbonyldiimidazole (1.07 g, 0.007 mol) and (2-aminomethyl-3-fluoro-5-nitrophenyl)methanol (1.1g, 0.0055 mol) in acetonitrile (20 mL) are heated at 60 °C for 6 hours. The reaction mixture is diluted with water and extracted with dichloromethane. The organic layer is separated, washed with water and brine, dried (Na₂SO₄) and evaporated. The residue is purified by flash chromatography to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 4.48 min; MS (m/z): (M-H)⁺ 225.0. Step 6: Preparation of 6-amino-8-fluoro-1-methyl-1H-benzo[d][1,3]oxazin-2(4H)-one.

Iron powder (0.63 g, 0.012 mol) is added portionwise to 8-fluoro-1-methyl-6-nitro-1H-benzo[d][1,3]oxazin-2(4H)-one (0.65 g, 0.003 mol) and ammonium chloride (1.52 g, 0.029 mol) in ethanol (20 mL) and water (10 mL) at 90 °C. The reaction mixture is stirred vigorously and heated for 1 hour, allowed to cool to room temperature, and diluted with dichloromethane (500 mL). The mixture is filtered through celite, washed with water and brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 2.22 min; MS (m/z): (M+H)⁺ 197.1.

### Step 7: Preparation of (R)-methyl 3-(8-fluoro-1-methyl-2-oxo-2,4-dihydro-1H-benzo[d][1,3]oxazin-6-ylamino)-2-hydroxypropanoate.

6-Amino-8-fluoro-1-methyl-1H-benzo[d][1,3]oxazin-2(4H)-one (0.54 g, 0.0023 mol), (R)-methyl oxirane-2-carboxylate (0.26g, 0.0023 mol) and lithium trifluoromethanesulfonate(0.398 g, 0.0023 mol) in acetonitrile are heated at 70 °C for 12 hours. The reaction is diluted with ethyl acetate, washed with water and brine, dried (Na₂SO₄) and evaporated. The residue is purified by flash chromatography to give the title compound. (SYMMETRY C₁₈ 3.5 µM, 4.6 x 30 mm column; gradient elution 2%-98% MeCN with 0.1 % TFA over 10 min; 2 mL/min rate): retention time = 3.602 min; MS (m/z): (M+H)⁺ 299.2.

### Step 8: Preparation of (R)-methyl 3-(8-fluoro-1-methyl-2-oxo-2,4-dihydro-1H-benzo[d][1,3]oxazin-6-yl)-2-oxooxazolidine-5-carboxylate.

(R)-Methyl 3-(8-fluoro-1-methyl-2-oxo-2,4-dihydro-1H-benzo[d][1,3]oxazin-6-ylamino)-2-hydroxypropanoate (0.60 g, 0.0021 mol) and 1,1-carbonyldiimidazole (0.417 g, 0.0026 mol) in acetonitrile (5 mL) are stirred and heated at 60 °C for 12 hours. The reaction is diluted with ethyl acetate, washed with water and brine, dried (Na₂SO₄) and evaporated. The residue is purified by flash chromatography to provide the title compound. HPLC (SYMMETRY C₁₈ 3.5 µM, 4.6 x 30 mm column; gradient elution 2%-98% MeCN with 0.1% TFA over 10 min; 2 mL/min rate): retention time = 4.201min; MS (m/z): (M+H)⁺ 325.1. Step 9: Preparation of (*R*)-3-(8-fluoro-1-methyl-2-oxo-2,4-dihydro-1H-benzo[d][1,3]oxazin-6-yl)-2-oxooxazolidine-5-carboxamide.

Ammonia in methanol (4 mL, 2M solution) is added to (*R*)-methyl 3-(8-fluoro-1-methyl-2-oxo-2,4-dihydro-1H-benzo[d][1,3]oxazin-6-yl)-2-oxooxazolidine-5-carboxylate (0.2 g, 0.00062 mol) at 0 °C and stirred for 2 hours. The reaction is evaporated and the residue triturated with methanol to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.33 min; ¹H NMR (300 MHz, DMSO₃-*d*₆) 7.54 (dd, *J* = 12.9, 2.5 Hz, 1H), 7.36(d, *J* = 1.6 Hz, 1H), 5.22 (s, 2H), 5.04 (q, *J* = 5.8, 3.9 Hz, 1H), 4.24 (t, *J* = 9.3 Hz, 1H), 3.99(q, *J* = 5.8, 3.3 Hz, 1H), 3.36 (d, *J* = 6.0 Hz, 3H); MS for C₁₃H₁₂FN₃O₅ m/z 310.2 (M+H)⁺.

### Example 9 Preparation of (R)-3-(8-fluoro-1-methyl-2-oxo-2,4-dihydro-1H-benzo[d][1,3]oxazin-6-yl)-N-methyl-2-oxooxazolidine-5-carboxamide

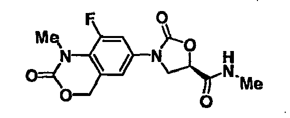

Methylamine in methanol (4 mL, 2M solution) is added to (*R*)-methyl 3-(8-fluoro-1-methyl-2-oxo-2,4-dihydro-1H-benzo[d][1,3]oxazin-6-yl)-2-oxooxazolidine-5-carboxylate (0.2 g, 0.00062 mol) at 0 °C and stirred for 2 hours. The reaction is evaporated and the residue triturated with methanol to give the title compound. HPLC (SYMMETRY C₁₈ 3.5 µM, 4.6 x 30 mm column; gradient elution 2%-98% MeCN with 0.1 % TFA over 10 min; 2 mL/min rate): retention time = 3.49 min; ¹H NMR (300 MHz, DMSO₃-*d₆*) 7.53 (dd, *J* = 12.6, 2.5 Hz, 1H), 7.36 (d, *J* = 1.6 Hz, 1H), 5.22 (s, 2H), 5.07 (q, *J* = 5.8, 3.9 Hz, 1H), 4.24 (t, *J* = 9.3 Hz, 1H), 4.0 (q, *J* = 5.8, 3.3 Hz, 1H), 3.36 (d, *J* = 6.0 Hz, 3H), 2.65 (d, *J* = 4.7 Hz, 3H); MS for C₁₄H₁₄FN₃O₅ m/z 324.2 (M+H)⁺.

### Example 10 Preparation of (S)-N-((3-(8-fluoro-1-methyl-2-oxo-2,4-dihydro-1H-benzo[d][1,3]oxazin-6-yl)-2-oxooxazolidin-5-yl)methyl)acetamide

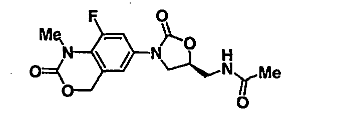

### Step 1: Preparation of benzyl 8-fluoro-1-methyl-2-oxo-2,4-dihydro-1H-benzo[d][1,3]oxazin-6-ylcarbamate.

Benzyl chloroformate (0.72 mL, 0.0051 mol) is added dropwise to 6-amino-8-fluoro-1-methyl-1H-benzo[d][1,3]oxazin-2(4H)-one (0.9 g, 0.005 mol) and pyridine (0.73 mL, 0.010 mol) in dichloromethane (15 mL) at 0 °C. The mixture is stirred at 0 °C for 30 min, allowed to warm to room temperature and then diulted with water. The organic layer is separated, washed with brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 5.489 min; MS (m/z): (M+H)⁺ 331.1

### Step 2: Preparation of (S)-tert-butyl (3-(8-fluoro-1-methyl-2-oxo-2,4-dihydro-1H-benzo[d][1,3]oxazin-6-yl)-2-oxooxazolidin-5-yl)methylcarbamate

Lithium *t*-butoxide (1.0 M solution in THF, 8.7 mL, 0.009 mol) is added dropwise at 0 °C to benzyl 8-fluoro-1-methyl-2-oxo-2,4-dihydro-1H-benzo[d][1,3]oxazin-6-ylcarbamate (1.2g, 0.004 mol) and (3-chloro-2-hydroxy-propyl)-carbamic acid *tert*-butyl ester (0.91 g, 0.0045 mmol) in DMF (10 mL). The mixture is allowed to warm to room temperature and stirred for 14 h. The reaction is quenched with saturated aqueous ammonium chloride, diluted with water and extracted with dichloromethane. The organic layer is washed with brine, dried (Na₂SO₄) and evaporated. The residue is purified by flash column chromatography (20% EtOAc/Hexane) to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 4.817 min; MS (m/z): (M+H)⁺ 396.3.

### Step 3: Preparation of (S)-6-(5-(aminomethyl)-2-oxooxazolidin-3-yl)-8-fluoro-1-methyl-1H-benzo[d][1,3]oxazin-2(4H)-one.

(S)-tert-Butyl (3-(8-fluoro-1-methyl-2-oxo-2,4-dihydro-1H-benzo[d][1,3]oxazin-6-yl)-2-oxooxazolidin-5-yl)methylcarbamate (0.6 g, 0.0022 mol) is stirred with 50% TFA/DCM (4 mL) for 1 h at room temperature. The reaction mixture is evaporated to give the title compound as the TFA salt (0.55 g, 97%); (SYMMETRY C₁₈ 3.5 µM, 4.6 x 30 mm column; gradient elution 2%-98% MeCN with 0.1 % TFA over 10 min; 2 mL/min rate): retention time = 2.869 min; MS (m/z): (M+H)⁺ 296.1.

### Step 4. Preparation of (S)-N-((3-(8-fluoro-1-methyl-2-oxo-2,4-dihydro-1H-benzo[d][1,3]oxazin-6-yl)-2-oxooxazolidin-5-yl)methyl)acetamide.

Acetic anhydride (0.113 mL, 0.0011 mmol) is added dropwise at 0 °C to (S)-6-(5-(aminomethyl)-2-oxooxazolidin-3-yl)-8-fluoro-1-methyl-1H-benzo[d][1,3]oxazin-2(4H)-one (0.30 g, 0.00102 mol) and pyridine (0.24 mL, 0.0034 mol) in dichloromethane (5 mL). The reaction mixture is stirred at 0 °C for 30 min then allowed to warm to room temperature. The reaction mixture is diluted with dichloromethane washed with water, citric acid and brine, dried (Na₂SO₄) and evaporated. The residue is purified by PTLC (10% MeOH/DCM) to give the title compound (0.15 g, 48%); (SYMMETRY C₁₈ 3.5 µM, 4.6 x 30 mm column; gradient elution 2%-98% MeCN with 0.1% TFA over 10 min; 2 mL/min rate): retention time = 3.456 min; ¹H NMR (300 MHz, DMSO₃-*d₆*) 7.50 (dd, *J* = 12.8, 2.6 Hz, 1H), 7.29(d, *J* = 1.7 Hz, 1H), 5.22 (s, 2H), 4.74 (m, 1H), 4.09 (t, *J* = 9.0 Hz, 1H), 3.71 (q, *J* = 6.3, 2.8 Hz, 1H), 3.41 (q, *J* = 5.5, 5.1 Hz, 2H), 3.36 (d, *J* = 6.0 Hz, 3H), 1.83 (s, 3H); MS for C₁₅H₁₆FN₃O₅ m/z 338.1 (M+H)⁺.

### Example 11 Preparation of (S)-N-((3-(8-fluoro-1-methyl-2-oxo-2,4-dihydro-1H-benzo[d][1,3]oxazin-6-yl)-2-oxooxazolidin-5-yl)methyl)propionamide

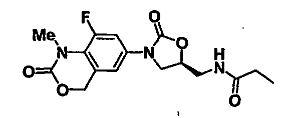

Propionic anhydride (0.145 mL, 0.0011 mmol) is added dropwise at 0 °C to (S)-6-(5-(aminomethyl)-2-oxooxazolidin-3-yl)-8-fluoro-l-methyl-1H-benzo[d][1,3]oxazin-2(4H)-one (0.30 g, 0.00102 mol) and pyridine (0.24 mL, 0.0034 mol) in dichloromethane (5 mL). The reaction mixture is stirred at 0 °C for 30 min then allowed to warm to room temperature. The reaction mixture is diluted with dichloromethane washed with water, citric acid and brine, dried (Na₂SO₄) and evaporated. The residue is purified by PTLC (10% MeOH/DCM) to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.685 min; ¹H NMR (300 MHz, DMSO₃-*d₆*) 7.51 (dd, *J* = 12.9, 2.5 Hz, 1H), 7.29 (d, *J* = 1.7 Hz, 1H), 5.23 (s, 2H), 4.73 (m, 1H), 4.08 (t, *J* = 9.1 Hz, 1H), 3.71 (q, *J* = 6.3, 2.8 Hz, 1H), 3.41 (q, *J* = 5.5, 5.1 Hz, 2H), 3.36 (d, *J* = 6.0 Hz, 3H), 2.08 (q, *J* = 7.4, 7.7 Hz, 2H), 0.94 (t, *J* = 5.8 Hz, 3H); MS for C₁₆H₁₈FN₃O₅ m/z 352.1 (M+H)⁺.

### Example 12 Preparation of (S)-methyl (3-(8-fluoro-1-methyl-2-oxo-2,4-dihydro-1H-benzo[d][1,3]oxazin-6-yl)-2-oxooxazolidin-5-yl)methylcarbamate

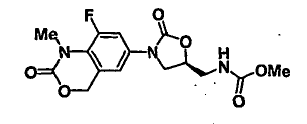

Methyl chloroformate (0.06 ml, 0.0008 mol) is added dropwise at 0 °C (*S*)-6-(5-(aminomethyl)-2-oxooxazolidin-3-yl)-8-fluoro-1-methyl-1H-benzo[d][1,3]oxazin-2(4H)-one (0.2 g, 0.0006 mol) and N,N-diisopropylethylamine (0.22 ml, 0.00013 mol) in dichloromethane (5 ml). The mixture is stirred at 0 °C for 30 minutes and then allowed to warm to room temperature. The reaction is diluted with dichloromethane, washed with water, citric acid and brine, dried (Na₂SO₄) and evaporated. The residue is purified by PTLC (10% MeOH/DCM) to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.805 min; ¹H NMR (300 MHz, DMSO₃-*d₆*) 7.50 (dd, *J* = 12.8, 2.6 Hz, 1H), 7.29(d, *J* = 1.7 Hz, 1H), 5.23 (s, 2H), 4.72 (m, 1H), 4.09 (t, *J* = 9.0 Hz, 1H), 3.71 (dd, *J* = 6.3, 2.8 Hz, 1H), 3.53 (s, 3H), 3.41 (q, *J* = 5.5, 5.1 Hz, 2H), 3.36 (d, *J =* 6.0 Hz, 3H); MS for C₁₅H₁₆FN₃O₆ m/z 354.2.

### Example 13 Preparation of (S)-N-((3-(5,8-difluoro-1-methyl-2-oxo-2,4-dihydro-1H-benzo[d][1,3]oxazin-6-yl)-2-oxooxazolidin-5-yl)methyl)acetamide

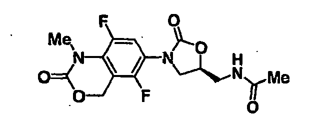

### Step 1: Preparation of (2,3,6-trifluorophenyl)methanol.

Sodium borohydride (2.411 g, 0.064 mol) is added at 0 °C to 2,3,6-trifluorobenzaldehyde (8.5 g, 0.053 mol) in methanol (100 mL). The mixture is stirred at 0 °C for 30 min and then quenched by adding ice. Methanol is removed under vacuum and the residue diluted with dichloromethane. The organic layer is separated, washed with water, citric acid and brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.667 min; MS (m/z): (M+H)⁺ 163.2

### Step 2: Preparation of 2, 3, 6-trifluorobenzyl acetate.

Acetic anhydride (5.48 mL, 0.054 mol) is added to (2,3,6-trifluorophenyl)methanol (8.0 g, 0.049 mol) and pyridine (5.86 mL, 0.074 mol) in dichloromethane (100 mL) and the mixture stirred overnight at room temperature. The reaction is diluted with 2N aqueous hydrochloric acid, the organic layer separated, washed with water and brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 5.152 min; MS (m/z): (M+H)⁺ 205.1

### Step 3: Preparation of 2,3,6-trifluoro-5-nitrobenzyl acetate.

2,3,6-trifluorobenzyl acetate (9.0 g, 0.044 mol) is added dropwise to fuming nitric acid (40 mL) at -10 °C and stirred at that temperature for 1 hour. The reaction mixture is then poured into ice water and extracted with dichloromethane. The organic layer is separated, washed with water and brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 5.232; MS (m/z): (M-H)⁺ 248.0.

### Step 4: Preparation of (2,3,6-trifluoro-5-nitrophenyl)methanol.

Hydrochloric acid (6N, 25 mL) and 2,3,6-trifluoro-5-nitrobenzyl acetate (5.0 g, 0.020mol) are heated at 60 °C for 3 hours. The mixture is adjusted to pH = 8 with aqueous sodium bicarbonate and extracted with ethyl acetate. The organic layer is washed with water and brine, dried (Na₂S0₄) and evaporated to give the title compound. HPLC (SYMMETRY C₁₈ 3.5 µM, 4.6 x 30 mm column; gradient elution 2%-98% MeCN with 0.1% TFA over 10 min; 2 nL/min rate): retention time = 3.890 min; MS (m/z): (M-H)⁺ 206.1.

### Step 5: Preparation of (2,5-difluoro-6-(methylamino)-3-nitrophenyl)methanol.

Methylamine (40% by weight in water, 1.32 mL, 0.021mol) and (2,3,6-trifluoro-5-nitrophenyl)methanol (2.2g, 0.011 mol) in dimethylsulfoxide (20 mL) are heated at 100 °C for 30 minutes. The reaction mixture is diluted with water and extracted with dichloromethane. The organic layer is separated, washed with water and brine, dried (Na₂SO₄) and evaporated to give the title compound (1.58g, 68 %); (SYMMETRY C₁₈ 3.5 µM, 4.6 x 30 mm column; gradient elution 2%-98% MeCN with 0.1% TFA over 10 min; 2 mL/min rate): retention time = 4.483; MS (m/z): (M-H)⁺217.0.

### Step 6: Preparation of 5,8-difluoro-1-methyl-6-nitro-1H-benzo[d][1,3]oxazin-2(4H)-one.

1,1-Carbonyl diimidazole (1.41 g, 0.009 mol) and (2,5-difluoro-6-(methylamino)-3-nitrophenyl)methanol (1.58 g, 0.0073 mol) in acetonitrile (20 mL) are heated at 60 °C for 6 hours. The reaction mixture is diluted with water and the resulting precipitate collected and dried under vacuum the title compound. HPLC (SYMMETRY C₁₈ 3.5 µM, 4.6 x 30 mm column; gradient elution 2%-98% MeCN with 0.1% TFA over 10 min; 2 mL/min rate): retention time = 4.77 min; MS (m/z): (M-H)⁺ 243.0.

### Step 7: Preparation of 6-amino-5,8-difluoro-1-methyl-1H-benzo[d][1,3]oxazin-2(4H)-one.

Iron powder (0.81 g, 0.015 mol) is added portionwise to 5,8-difluoro-1-methyl-6-nitro-1H-benzo[d][1,3]oxazin-2(4H)-one (0.9 g, 0.004 mol) and ammonium chloride (1.95 g, 0.037 mol) in ethanol (20 mL) and water (10 mL) at 90 °C. The reaction mixture is stirred vigorously and heated for 1 hour, allowed to cool to room temperature, and diluted with dichloromethane (500 mL). The mixture is filtered through celite, washed with water and brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.25 min; MS (m/z): (M+H)⁺ 214.9.

### Step 8: Preparation of benzyl 5,8-difluoro-1-methyl-2-oxo-2,4-dihydro-1H-benzo[d][1,3]oxazin-6-ylcarbamate.

Benzyl chloroformate (0.51 mL, 0.004 mol) is added dropwise to a mixture of 6-amino-5,8-difluoro-1-methyl-1H-benzo[d][1,3]oxazin-2(4H)-one (0.7 g, 0.0033 mol) and pyridine (0.52 mL, 0.0065 mol) in dichloromethane (15 mL) at 0 °C. The reaction mixture is stirred at 0 °C for 30 min, allowed to warm at room temperature and then diluted with water. The organic layer is separated, washed with 2N hydrochloric acid, and brine, dried (Na₂SO₄) and evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 5.616 min; MS (m/z): (M+H)⁺ 348.1.

### Step 9: Preparation of (S)-tert-butyl (3-(5,8-difluoro-1-methyl-2-oxo-2,4-dihydro-1H-benzo[d][1,3]oxazin-6-yl)-2-oxooxazolidin-5-yl)methylcarbamate.

Lithium *t*-butoxide (1.0 M solution in THF, 6.2 mL, 0.006 mol) is added dropwise at 0 °C to benzyl 5,8-difluoro-1-methyl-2-oxo-2,4-dihydro-1H-benzo[d][1,3]oxazin-6-ylcarbamate (0.9 g, 0.0025 mol) and (3-chloro-2-hydroxy-propyl)-carbamic acid *tert*-butyl ester (0.65 g, 0.0031 mol) in DMF (10 mL). The mixture is allowed to warm to room temperature and stirred for 14 hours. The reaction is quenched with saturated aqueous ammonium chloride, diluted with water and extracted with dichloromethane. The organic layer is washed with brine, dried (Na₂SO₄) and evaporated. The residue is purified by flash column chromatography (20% EtOAc/Hexane) to give the title compound. HPLC (SYMMETRY C₁₈ 3.5 µM, 4.6 x 30 mm column; gradient elution 2%-98% MeCN with 0.1 % TFA over 10 min; 2 mL/min rate): retention time = 4.927 min; MS (m/z): (M+H)⁺ 396.3.

### Step 10: Preparation of (S)-6-(5-(aminomethyl)-2-oxooxazolidin-3-yl)-5,8-difluoro-1-methyl-1H-benzo[d][1,3]oxazin-2(4H)-one.

(S)-tert-butyl (3-(5,8-difluoro-1-methyl-2-oxo-2,4-dihydro-1H-benzo[d][1,3]oxazin-6-yl)-2-oxooxazolidin-5-yl)methylcarbamate (0.6 g, 0.0022 mol) is stirred with 50% TFA/DCM (4 mL) for 1 h at room temperature. The reaction mixture is evaporated to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 2.751 min; MS (m/z): (M+H)⁺ 314.0.

### Step 11: Preparation of (S)-N-((3-(5,8-difluoro-1-methyl-2-oxo-2,4-dihydro-1H-benzo[d][1,3]oxazin-6-yl)-2-oxooxazolidin-5-yl)methyl)acetamide.

Acetic anhydride (0.113 mL, 0.0011 mmol) is added dropwise at 0 °C to (S)-6-(5-(aminomethyl)-2-oxooxazolidin-3-yl)-8-fluoro-1-methyl-1H-benzo[d][1,3]oxazin-2(4H)-one (0.30 g, 0.00102 mol) and pyridine (0.24 mL, 0.0034 mol) in dichloromethane (5 mL). The reaction mixture is stirred at 0 °C for 30 min then allowed to warm to room temperature. The reaction mixture is diluted with dichloromethane washed with water, citric acid and brine, dried (Na₂SO₄) and evaporated. The residue is purified by preparative TLC (10% MeOH/DCM) to give product (0.15 g, 48%); (SYMMETRY C₁₈ 3.5 µM, 4.6 x 30 mm column; gradient elution 2%-98% MeCN with 0.1% TFA over 10 min; 2 mL/min rate): retention time = 3.254 min; ¹H NMR (300 MHz, DMSO₃-*d₆*) 7.55 (m, 1H), 5.32 (s, 2H), 4.74 (m, 1H), 4.04 (t, *J* = 8.2 Hz, 1H), 3.68 (q, *J* = 6.0, 2.5 Hz, 1H), 3.41 (q, *J* = 5.2, 4.9 Hz, 2H), 3.36 (d, *J* =6.0 Hz, 3H), 1.83 (s, 3H); MS for C₁₅H₁₅F₂N₃O₅ m/z 356.4 (M+H)⁺.

### Example 14 Preparation of (S)-methyl (3-(5,8-difluoro-1-methyl-2-oxo-2,4-dihydro-1H-benzo[d][1,3]oxazin-6-yl)-2-oxooxazolidin-5-yl)methylcarbamate

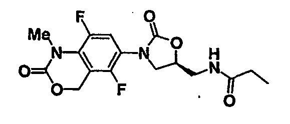

Propionic anhydride (0.145 mL, 0.0011 mmol) is added dropwise at 0 °C to (S)-6-(5-(aminomethyl)-2-oxooxazolidin-3-yl)-8-fluoro-1-methyl-1H-benzo[d][1,3]oxazin-2(4H)-one (0.30 g, 0.00102 mol) and pyridine (0.24 mL, 0.0034 mol) in dichloromethane (5 mL). The reaction mixture is stirred at 0 °C for 30 min., then allowed to warm to room temperature. The reaction mixture is diluted with dichloromethane washed with water, citric acid and brine, dried (Na₂SO₄) and evaporated. The residue is purified by PTLC (10% MeOH/DCM) to give the title compound. HPLC (SYMMETRY C₁₈ 3.5 µM, 4.6 x 30 mm column; gradient elution 2%-98% MeCN with 0.1% TFA over 10 min; 2 mL/min rate): retention time = 3.511 min; ¹H NMR (300 MHz, DMSO₃-*d₆*) 7.54 (m, 1H), 5.32 (s, 2H), 4.75 (m, 1H), 4.04 (t, *J* = 8.2 Hz, 1H), 3.69 (q, *J* = 6.0, 2.5 Hz, 1H), 3.41 (q, *J* = 5.2, 4.9 Hz, 2H), 3.36 (d, *J* = 6.6 Hz, 3H), 2.11 (q, *J* = 7.4, 7.7 Hz, 2H), 0.98 (t, *J* = 7.4 Hz, 3H); MS for C₁₅H₁₅F₂N₃O₆ m/z 370.4 (M+H)⁺.

### Example 15 Preparation of N-{[(5S)-3-(9-fluoro-1-methyl-2-oxo-1,2,4,5-tetrahydro-3,1-benzoxazepin-7-yl)-2-oxo-1,3-oxazolidin-5-yl]methyl} acetamide

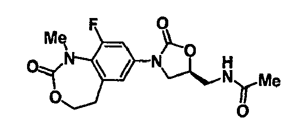

### Step 1: Preparation of {2-[benzyl(methyl)amino]-3-fluoro-5-nitrophenyl} acetic acid.

To a cold (-10°C) solution of (2,3-difluorophenyl)acetic acid (40.0 g, 232 mmol) in concentrated sulfuric acid (120 mL) is added a cold mixture (0°C) of nitric acid (21 mL, 70% aqueous, 0.33 mol) and concentrated sulfuric acid (40 mL) over 5 min. After 20 min, the mixture is poured into ice water (1 L) and extracted with CH₂Cl₂ (3x300 mL). The combined organic layers are washed by brine, dried (Na₂SO₄), filtered and concentrated to provide a mixture of (2,3-difluoro-5-nitrophenyl)acetic acid and (2,3-difluoro-6-nitrophenyl)acetic acid (95%). This mixture is directly used in the next step without further purification. To a solution of the above regioisomeric mixture (52 g, 0.24 mol) in DMSO (100 mL) is added N-benzylmethylamine (175 mL, 1.36 mol). The mixture is stirred at 80°C for 17 h, cooled to 23°C, diluted with aqueous sodium hydroxide (30 g, in 1 L H₂O) and extracted with ether (3x300 mL). The aqueous layer is then acidified with 12 M aqueous HCl to pH = 4 and extracted with CH₂Cl₂ (4x300 mL). The combined CH₂Cl₂ extracts are washed by brine, dried (Na₂SO₄), filtered and concentrated to afford the title compound. ¹H NMR (300 MHz, CDCl₃): 2.64 (s, 1H), 2.70 (s, 3H), 3.82 (s, 2H), 4.15 (d, *J* =1.8 Hz, 2H), 7.27 (m, 5H), 7.90 (dd, *J* =2.7 Hz, *J* =11.4 Hz, 1H), 7.95 (m, 1H).

### Step 2. Preparation of 2-{2-[benzyl(methyl)amino]-3-fluoro-5-nitrophenyl}ethanol.

To a cold solution of {2-[benzyl(methyl)amino]-3-fluoro-5-nitrophenyl}acetic acid (12.5 g, 39.3 mmol) in THF (100 mL) at -25°C is added BH₃•SMe₂ (9.6 mL, 101 mmol). The mixture is allowed to warm to 23°C, stirred for 17 h, and then treated with aqueous NaOH (3.0 M, 70 mL) and heated at reflux for 1.3 h. The mixture is cooled down to 23°C, neutralized with 1.0 *M* HCl aqueous to pH = 7 and extracted with EtOAc (3x300 mL). The combined organic layers are washed by brine, dried (Na₂SO₄), filtered and concentrated. The crude product is purified by chromatography on a silica gel column, eluting with a gradient increasing in polarity from 5% to 25% ethyl acetate in hexane. Relevant fractions are combined to give the title compound. ¹H NMR(300 MHz, CDCl₃): 1.81 (bs, 1H), 2.76 (d, *J* =2.1 Hz, 3H), 3.08 (t, *J* =6.6 Hz, 2H), 3.89 (m, 2H), 4.20 (d, *J* =1.5 Hz, 2H), 7.31 (m, 5H), 7.81 (dd, *J* =2.7 Hz, J =12.0 Hz, 1H), 7.97 (m, 1H).

### Step 3. Preparation of N-benzyl-N-{2-fluoro-6-[2-(tert-butyldimethylsilyloxy)ethyl]-4-nitrophenyl}-N-methylamine.

To a solution of 2-{2-[benzyl(methyl)amino]-3-fluoro-5-nitrophenyl}ethanol (11.1 g, 36.5 mmol) in DMF (60 mL) at 23°C is added imidazole (4.96 g, 73 mmol) and tert-butyldimethylsilyl chloride (6.6 g, 43.8 mmol). The reaction mixture is stirred at 23°C for 17 h, diluted with saturated aqueous NH₄Cl (500 mL) and extracted with CH₂Cl₂ (3 x 200 mL). The combined organic layers are washed by brine, dried (Na₂SO₄), filtered and concentrated. The crude product is purified by chromatography on a silica gel column, eluting with a gradient increasing in polarity from 0% to 10% ethyl acetate in hexane. Relevant fractions are combined and concentrated to afford the title compound. ¹H NMR (300 MHz, CDCl₃): 0.01 (s, 6H), 0.85 (s, 9H), 2.73 (d, *J* =1.8 Hz, 3H), 3.05 (t, *J* =6.6 Hz, 2H), 3.86 (t, *J* =6.6 Hz, 2H), 4.20 (d, *J* =2.1 Hz, 2H), 7.32 (m, 5H), 7.79 (dd, *J* =2.7 Hz, *J* =11.7 Hz, 1H), 8.02 (t, *J* =1.5 Hz, 1H).

### Step 4. Preparation of N-{4-amino-2-fluoro-6-[2-(tert-butyldimethylsilyloxy)ethyl]phenyl}-N-benzyl-N-methylamine.

To a solid mixture of N-benzyl-N-{2-fluoro-6-[2-(tert-butyldimethylsilyloxy)ethyl]-4-nitrophenyl}-N-methylamine (15.0 g 36.5 mmol) and NH₄Cl (21.5 g, 21.5 mol) is added EtOH (230 mL) and H₂O (115 mL). The reaction mixture is heated to reflux, and iron powder (7.0 g, 125 mmol) is added in three portion over 15 min. The resulting mixture is heated for 1h, cooled to 23°C, diluted with CH₂Cl₂ (500 mL) and filtered through celite. The layers are separated and the organic phase washed with water and brine, and dried (Na₂SO₄), filtered and concentrated afford the title compound which is used in the next step without further purification. ¹H NMR (300 MHz, CDCl₃): 0.01 (s, 6H), 0.89 (s, 9H), 2.60 (d, *J* =0.9 Hz, 3H), 2.95 (bs, 2H), 3.58 (s, 2H), 3.74 (t, *J* =5.4 Hz, 2H), 4.07 (s, 2H), 6.26 (dd, *J* =1.8 Hz, *J* =9.9 Hz, 1H), 6.30 (m, 1H), 7.28 (m, 5H).

### Step 5. Preparation of benzyl 4-[benzyl(methyl)amino]-3-fluoro-5-[2-(tert-butyldimethylsilyloxy)ethyl] phenylcarbamate.

To a solution ofN-{4-amino-2-fluoro-6-[2-(tert-butyldimethylsilyloxy)ethyl]phenyl}-N-benzyl-N-methylamine (14.2 g, 36.5 mmol) in CH₂Cl₂ (400 mL) at 23°C is added pyridine (5.0 mL, 61.8 mmol) and CbzCl (5.55 mL, 38.9 mmol). After 1h, the reaction mixture is diluted with CH₂Cl₂ (300 mL), washed by NH₄Cl aqueous, brine, dried (Na₂SO₄), filtered and concentrated. The crude product is purified by chromatography on a silica gel column, eluting with a gradient increasing in polarity from 0% to 10% ethyl acetate in hexane. Relevant fractions are combined and concentrated to afford the title compound. ¹H NMR (300 MHz, CDCl₃): 0.07 (s, 6H), 0.86 (s, 9H), 2.62 (d, *J* =1.5 Hz, 3H), 2.97 (t, *J* =7.2 Hz, 2H), 3.74 (t, *J* =7.2 Hz, 2H), 4.09 (s, 2H), 5.20 (s, 2H), 6.57 (s, 1H), 6.78 (d, *J* =1.5 Hz, 1H), 7.19-7.42 (m, 11H).

### Step 6. Preparation of tert-butyl ((5S)-3-{4-[benzyl(methyl)amino]-3-fluoro-5-[2-(tert-butyldimethylsilyloxy)ethyl]phenyl}-2-oxo-1,3-oxazolidin-5-yl)methylcarbamate.

Lithium *t*-butoxide solution (92.6 mL, of a 1.0 M THF solution, 92.6 mmol) is added to a cooled (0°C) solution of benzyl 4-[benzyl(methyl)amino]-3-fluoro-5-[2-(tert-butyldimethylsilyloxy)ethyl] phenylcarbamate (17.3 g, 33 mmol) in DMF (15 mL). Solid tert-butyl (2S)-3-chloro-2-hydroxypropylcarbamate (13 g, 62 mmol) is then added and the solution is allowed to warm to 23°C and stirred for 20 h. Saturated NH₄Cl aqueous solution (400 mL) is then added and the solution is extracted with CH₂Cl₂ (3x300 mL). The combined organic phases are dried (Na₂SO₄), filtered and concentrated. The crude product is purified by chromatography on a silica gel column, eluting with a gradient increasing in polarity from 0% to 25% ethyl acetate in hexane. Relevant fractions are combined and concentrated to afford the title compound. ¹H NMR (300 MHz, CDCl₃): 0.01 (s, 6H), 0.87 (s, 9H), 1.41 (s, 9H), 2.63 (s, 3H), 3.00 (t, *J* =7.2 Hz, 2H), 3.48 (m, 2H), 3.77 (m, 3H), 3.99 (t, *J* =9.0 Hz, 1H), 4.11 (m, 2H), 4.72 (m, 1H), 4.97 (m, 1H), 6.97 (m, 1H), 7.21-7.42 (m, 6H).

### Step 7. Preparation of tert-butyl {(5S)-3-[4-[benzyl(methyl)amino]-3-fluoro-5-(2-hydroxyethyl)phenyl]-2-oxo-1,3-oxazolidin-5-yl}methylcarbamate.

To a solution of tert-butyl ((5S)-3-{4-[benzyl(methyl)amino]-3-fluoro-5-[2-(tert-butyldimethylsilyloxy)ethyl]phenyl}-2-oxo-1,3-oxazolidin-5-yl)methylcarbamate (5.67 g, 9.66 mmol) in THF (42 mL) at 23°C is added Et₃N•3HF (4.68 mL, 28.7 mmol). The mixture is stirred for 17 h, diluted with ethyl acetate (500 mL), washed by saturated NH₄Cl aqueous, dried (Na₂SO₄), filtered and concentrated. The crude product is purified by chromatography on a silica gel column, eluting with a gradient increasing in polarity from 20% to 100% ethyl acetate in hexane. Relevant fractions are combined and concentrated to afford the title compound. ¹H NMR (300 MHz, DMSO-d₆): 1.34 (s, 9H), 2.58 (d, *J* =0.9 Hz, 3H), 2.89 (t, *J* =7.5 Hz, 2H), 3.25 (t, *J* =5.4 Hz, 2H), 3.56 (m, 2H), 3.75 (m, 1H), 3.96-4.10 (m, 3H), 4.67 (m, 2H), 7.08 (m, 1H), 7.20-7.37 (m, 7H).

### Step 8. Preparation of tert-butyl {(5S)-3-[3-fluoro-5-(2-hydroxyethyl)-4-(methylamino)phenyl]-2-oxo-1,3-oxazolidin-5-yl}methylcarbamate.

To a solution of tert-butyl {(5S)-3-[4-[benzyl(methyl)amino]-3-fluoro-5-(2-hydroxyethyl)phenyl]-2-oxo-1,3-oxazolidin-5-yl}methylcarbamate (3.7 g, 7.8 mmol) in MeOH (200 mL) at 23°C is added 20% Pd(OH)₂/C (2.0 g). The mixture is stirred under H₂ atmosphere for 17 h, filtered through Celite and concentrated to afford the title compound. This material is directly used in the next step without further purification (2.8 g, 94%); ¹H NMR (300 MHz, DMSO-d₆): 1.35 (s, 9H), 2.70 (t, *J* =6.9 Hz, 2H), 2.77 (d, *J* =3.6 Hz, 3H), 3.23 (t, *J* =5.4 Hz, 2H), 3.54 (t, *J* =6.9 Hz, 2H), 3.70 (dd, *J* =6.0 Hz, *J* =9.0 Hz, 1H), 4.01 (t, *J* =9.0 Hz, 1H), 4.53-4.67 (m, 3H), 6.94 (d, *J* =2.1 Hz, 1H), 7.24 (m, 2H).

### Step 9. Preparation of tert-butyl [(5S)-3-(9-fluoro-1-methyl-2-oxo-1,2,4,5-tetrahydro-3,1-benzoxazepin-7-yl)-2-oxo-1,3-oxazolidin-5-yl]methylcarbamate.

To a solid mixture of tert-butyl {(5S)-3-[3-fluoro-5-(2-hydroxyethyl)-4-(methylamino)phenyl]-2-oxo-1,3-oxazolidin-5-yl}methylcarbamate (1.4 g, 3.66 mmol) and 1,1'-carbonyldiimidazole (0.67 g, 4.14 mmol) at 23°C is added CH₃CN (28 mL). The mixture is stirred at 65°C for 3 h, cooled down to 23°C, and diluted with 300 mL of ethyl acetate. The organic phase is washed with saturated aqueous NH₄Cl, dried (Na₂SO₄), filtered and concentrated. The crude product is purified by chromatography on a silica gel column, eluting with a gradient increasing in polarity from 10% to 50% ethyl acetate in hexane. Relevant fractions are combined and concentrated to afford the title compound. ¹H NMR (300 MHz, DMSO-d₆): 1.35 (s, 9H), 2.82 (d, *J* =1.5 Hz, 3H), 2.92 (t, *J* =8.4 Hz, 2H), 3.23 (m, 4H), 3.70 (dd, *J* =6.0 Hz, *J* =9.0 Hz, 1H), 4.02 (t, *J* =9.0 Hz, 1H), 4.62 (m, 1H), 7.07-7.21 (m, 3H). Step 10: Preparation of N-{[(5S)-3-(9-fluoro-1-methyl-2-oxo-1,2,4,5-tetrahydro-3,1-benzoxazepin-7-yl)-2-oxo-1,3-oxazolidin-5-yl]methyl}acetamide.

Trifluoroacetic acid (5 mL) is added to a solution of tert-butyl [(5S)-3-(9-fluoro-1-methyl-2-oxo-1,2,4,5-tetrahydro-3,1-benzoxazepin-7-yl)-2-oxo-1,3-oxazolidin-5-yl]methylcarbamate (330 mg, 0.81 mmol) in CH₂Cl₂ (20 mL) at 23°C. The reaction mixture is stirred at 23°C for 60 min and then concentrated. The crude 7-[(5S)-5-(aminomethyl)-2-oxo-1,3-oxazolidin-3-yl]-9-fluoro-1-methyl-4,5-dihydro-3,1-benzoxazepin-2(1H)-one is used directly used in the next step. Acetic anhydride (0.1 mL, 1.08 mmol) and diisopropylethylamine (1.0 mL, 5.75 mmol) is added to a cooled (0°C) solution of crude 7-[(5S)-5-(aminomethyl)-2-oxo-1,3-oxazolidin-3-yl]-9-fluoro-1-methyl-4,5-dihydro-3,1-benzoxazepin-2(1H)-one (0.81 mmol) in CH₂Cl₂ (20 mL). The reaction mixture is stirred at 0°C for 30 min and then diluted with CHCl₃. The organic solution is washed with aqueous NaHCO₃, and dried (Na₂SO₄), filtered and concentrated. The crude product is purified by preparative HPLC to afford the title compound. HPLC (SYMMETRY C₁₈ 3.5 µM, 4.6 x 30 mm column; gradient elution 2%-98% MeCN with 0.1% TFA over 6 min; 2 mL/min rate): retention time = 1.704 min; ¹H NMR (300 MHz, DMSO-d₆): 1.82 (s, 3H), 2.82 (d, *J* =1.5 Hz, 3H), 2.93 (t, *J* =8.4 Hz, 2H), 3.24 (t, *J* =8.7 Hz, 2H), 3.37 (t, *J* =5.4 Hz, 2H), 3.64 (dd, *J* =6.6 Hz, *J* =9.3 Hz, 1H), 4.02 (t, *J* =9.0 Hz, 1H), 4.66 (m, 1H), 7.10 (m, 2H), 8.22 (t, *J* =6.0 Hz, 1H); MS (m/z): [M+H-CO₂]⁺ = 308.4.

### Example 16 Preparation of N-{[(5S)-3-(9-fluoro-1-methyl-2-oxo-1,2,4,5-tetrahydro-3,1-benzoxazepin-7-yl)-2-oxo-1,3-oxazolidin-5-yl]methyl}propanamide.

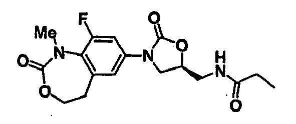

Trifluoroacetic acid (5 mL) is added to a solution of tert-butyl [(5S)-3-(9-fluoro-1-methyl-2-oxo-1,2,4,5-tetrahydro-3,1-benzoxazepin-7-yl)-2-oxo-1,3-oxazolidin-5-yl]methylcarbamate (330 mg, 0.81 mmol) in CH₂Cl₂ (20 mL) at 23°C. The reaction mixture is stirred at 23°C for 60 min and concentrated. The crude 7-[(5S)-5-(aminomethyl)-2-oxo-1,3-oxazolidin-3-yl]-9-fluoro-1-methyl-4,5-dihydro-3,1-benzoxazepin-2(1H)-oneobtained is used directly used in the next step. Propionic anhydride (0.11 mL, 0.85 mmol) and diisopropylethylamine (1.0 mL, 5.74 mmol) are added to a cooled (0°C) solution of crude 7-[(5S)-5-(aminomethyl)-2-oxo-1,3-oxazolidin-3-yl]-9-fluoro-1-methyl-4,5-dihydro-3,1-benzoxazepin-2(1H)-one (0.81 mmol) in CH₂Cl₂ (20 mL). The reaction mixture is stirred at 0°C for 30 min, and then diluted with CHCl₃. The organic solution is washed with aqueous NaHCO₃, and dried (Na₂SO₄), filtered and concentrated. The crude product is purified by preparative HPLC to afford the title compound. HPLC (SYMMETRY C₁₈ 3.5 µM, 4.6 x 30 mm column; gradient elution 2%-98% MeCN with 0.1% TFA over 6 min; 2 mL/min rate): retention time = 1.807 min; ¹H NMR (300 MHz, DMSO-*d₆*): 0.97 (t, *J* =7.8 Hz, 3H), 2.11 (q, *J* =7.8 Hz, 2H), 2.82 (d, *J* =1.5 Hz, 3H), 2.92 (t, *J* =8.4 Hz, 2H), 2.72 (t, *J* =8.7 Hz, 2H), 3.39 (m, 2H), 3.65 (dd, *J* =6.0 Hz, *J* =9.0 Hz, 1H), 4.02 (t, *J* =9.0 Hz, 1H), 4.66 (m, 1H), 7.11 (m, 2H), 8.14 (t, *J* =5.7 Hz, 1H); MS (m/z): [M+H-CO₂]⁺ = 322.0.

### Example 17 Preparation of methyl [(5S)-3-(9-fluoro-1-methyl-2-oxo-1,2,4,5-tetrahydro-3,1-benzoxazepin-7-yl)-2-oxo-1,3-oxazolidin-5-yl]methylcarbamate

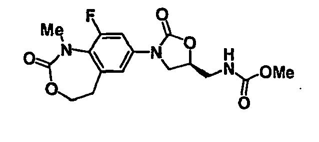

Trifluoroacetic acid (5 mL) is added to a solution of tert-butyl [(5S)-3-(9-fluoro-1-methyl-2-oxo-1,2,4,5-tetrahydro-3,1-benzoxazepin-7-yl)-2-oxo-1,3-oxazolidin-5-yl]methylcarbamate (330 mg, 0.81 mmol) in CH₂Cl₂ (20 mL) at 23°C. The reaction mixture is stirred at 23°C for 60 min and concentrated. The crude 7-[(5S)-5-(aminomethyl)-2-oxo-1,3-oxazolidin-3-yl]-9-fluoro-1-methyl-4,5-dihydro-3,1-benzoxazepin-2(1H)-one obtained is used directly used in the next step. Methyl chloroformate (0.08 mL, 1.04 mmol) and diisopropylethylamine (1.0 mL, 5.74 mmol) are added to a cooled (0°C) solution of crude 7-[(5S)-5-(aminomethyl)-2-oxo-1,3-oxazolidin-3-yl]-9-fluoro-1-methyl-4,5-dihydro-3,1-benzoxazepin-2(1H)-one (0.81 mmol) in CH₂Cl₂ (20 mL). The reaction mixture is stirred at 0°C for 30 min, and then diluted with CHCl₃. The organic solution is then washed with aqueous NaHCO₃, dried (Na₂SO₄), filtered and concentrated. The crude product is purified by preparative HPLC to afford the title compound. HPLC (SYMMETRY C₁₈ 3.5 µM, 4.6 x 30 mm column; gradient elution 2%-98% MeCN with 0.1 % TFA over 6 min; 2 mL/min rate): retention time = 2.008 min; ¹H NMR (300 MHz, DMSO-d₆): 2.82 (d, *J* =1.5 Hz, 3H), 2.93 (t, *J* =9.0 Hz, 2H), 3.25 (t, *J* =9.0 Hz, 2H), 3.30 (m, 2H), 3.53 (s, 3H), 3.68 (dd, *J* =6.3 Hz, *J* =9.3 Hz, 1H), 4.03 (t, *J* =9.0 Hz, 1H), 4.65 (m, 1H), 7.09 (m, 2H), 7.50 (t, *J* =6.0 Hz, 1H); MS (m/z): [M+H-CO₂]⁺ = 324.1.

### Example 18 Preparation of (5R)-3-(1-methyl-2-oxo-1,2,4,5-tetrahydro-3,1-benzoxazepin-7-yl)-2-oxo-1,3-oxazolidine-5-carboxamide.

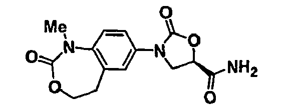

### Step 1. Preparation of 4,5-dihydro-3,1-benzoxazepin-2(1H)-one.

To a solid mixture of 2-(2-aminophenyl)ethanol (8.0 g, 58.3 mmol) and 1,1'-carbonyldiimidazole (10.4 g, 64.1 mmol) at 23°C is added CH₃CN (58 mL). The resulting mixture is stirred at 23°C for 1 h, concentrated, and diluted with CH₂Cl₂ (700 mL). The organic phase is washed with 0.5 M aqueous HCl, brine, and dried (Na₂SO₄), filtered, concentrated. The crude product is recrystallized from EtOAc/hexane to afford the title compound. ¹H NMR (300 MHz, DMSO-d₆): 3.09 (t, *J* =4.8 Hz, 2H), 4.36 (t, *J* =4.8 Hz, 2H), 6.92 (m, 1H), 7.05-7.15 (m, 3H), 9.49 (s, 1H).

### Step 2. Preparation of 1-methyl-4,5-dihydro-3,1-benzoxazepin-2(1H)-one.

To a solution of 4,5-dihydro-3,1-benzoxazepin-2(1H)-one (4.9 g, 30 mmol) in DMF (40 mL) at 0°C is added NaH (1.38 g, 60% in mineral oil, 34.5 mmol). The mixture stirred at 0°C for 20 min and the iodomethane (2.81 mL, 45 mmol) is added. The ice bath is removed and the mixture is stirred at 23°C for 30 min and diluted with CH₂Cl₂ (400 mL). The organic solution is washed with 0.5 M aqueous HCl, brine, and dried (Na₂SO₄), filtered, and concentrated. The crude product is purified by chromatography on a silica gel column, eluting with a gradient increasing in polarity from 5% to 25% ethyl acetate in hexane. Relevant fractions are combined to afford the title compound. ¹H NMR (300 MHz, DMSO-d₆): 2.95 (t, J = 6.6 Hz, 2H), 3.27 (s, 3H), 4.35 (t, J = 6.6 Hz, 2H), 7.19 (m, 1H), 7.30 (m, 3H).

### Step 3. Preparation of 1-methyl-7-nitro-4,5-dihydro-3,1-benzoxazepin-2(1H)-one.

To a cold (-10°C) solution of 1-methyl-4,5-dihydro-3,1-benzoxazepin-2(1H)-one (2.51 g, 14 mmol) in H₂SO₄ (5 mL) is added a cold mixture (0°C) of HNO₃ (1.2 mL, 70% aqueous, 19 mmol) and H₂SO₄ (10 mL) over 5 min. After 30 min, the mixture is poured into ice water (0.5 L) and extracted with CH₂Cl₂ (3x100 mL). The combined organic layers are washed with brine, and dried (Na₂SO₄), filtered and concentrated. The crude product is purified by chromatography on a silica gel column, eluting with a gradient increasing in polarity from 5% to 30% ethyl acetate in hexane. Relevant fractions are combined and concentrated to afford the title compound. ¹H NMR (300 MHz, DMSO-d₆): 3.14 (t, *J* = 6.6 Hz, 2H), 3.33 (s, 3H), 4.44 (t, *J* = 6.6 Hz, 2H), 7.55 (d, *J* = 9.0 Hz, 1H), 8.19 (dd, *J* = 2.7 Hz, *J* = 8.7 Hz, 1H), 8.27 (d, *J* = 2.7 Hz, 1H).

### Step 4. Preparation of 7-amino-1-methyl-4,5-dihydro-3,1-benzoxazepin-2(1H)-one.

To a solution of 1-methyl-7-nitro-4,5-dihydro-3,1-benzoxazepin-2(1H)-one (2.75 g, 12 mmol) in MeOH (320 mL) at 23°C is added 10% Pd/C (400 mg). The reaction mixture is stirred under a hydrogen atmosphere at 23°C for 17 h and then filtered through Celite with the aid of 20% MeOH/CH₂Cl₂. The filtrate is concentrated and the crude product recrystallized from EtOAc/hexane to afford the title compound. ¹H NMR (300 MHz, DMSO-d₆): 2.75 (t, *J* =6.6 Hz, 2H), 3.16 (s, 3H), 4.27 (t, *J* =6.6 Hz, 2H), 5.07 (s, 2H), 6.46 (m, 2H), 6.92 (d, *J* =8.4 Hz, 1H).

### Step 5. Preparation of methyl (2R)-2-hydroxy-3-[(1-methyl-2-oxo-1,2,4,5-tetrahydro-3,1-benzoxazepin-7-yl)amino]propanoate.

To a solution of 7-amino-1-methyl-4,5-dihydro-3,1-benzoxazepin-2(1H)-one (0.82 g, 4.3 mmol) in CH₃CN (20 mL) at 23°C is added LiOTf (738 mg, 4.73 mmol) and *R*-methyl glycidte (0.47 mL, 5.4 mmol). The solution is heated for 3 days at 70°C, and then cooled to 23°C and concentrated. The crude residue is purified by chromatography on a silica gel column, eluting with a gradient increasing in polarity from 30% to 70% ethyl acetate-hexanes. Relevant fractions are combined and concentrated to afford the title compound. ¹H NMR (300 MHz, DMSO-d₆): 2.80 (t, *J* = 6.6 Hz, 2H), 3.17 (s, 3H), 3.22 (t, *J* = 6.3 Hz, 1H), 3.36 (m, 1H), 3.62 (s, 3H), 4.23 (m, 1H), 4.28 (t, *J* = 6.6 Hz, 2H), 5.63 (t, *J* = 6.6 Hz, 1H), 5.68 (d, *J* = 6.0 Hz, 1H), 6.53 (m, 2H), 6.98 (m, 1H).

### Step 6. Preparation of methyl (5R)-3-(1-methyl-2-oxo-1,2,4,5-tetrahydro-3,1-benzoxazepin-7-yl)-2-oxo-1,3-oxazolidine-5-carboxylate.

To a solid mixture of methyl (2R)-2-hydroxy-3-[(1-methyl-2-oxo-1,2,4,5-tetrahydro-3,1-benzoxazepin-7-yl)amino]propanoate (1.1 g, 3.7 mmol) and 1,1'-carbonyldiimidazole (1.2 g, 7.4 mmol) at 23°C is added CH₃CN (15 mL). The resulting mixture is stirred at 45°C for 3 h, cooled to 23°C and concentrated. The residue is taken into ethyl acetate (300 mL) and washed with 0.5 M aqueous HCl, brine, and dried (Na₂SO₄), filtered, concentrated. The crude residue is purified by chromatography on a silica gel column, eluting with a gradient increasing in polarity from 60% to 90% ethyl acetate in hexane. Relevant fractions are combined and concentrated to afford the title compound. ¹H NMR (300 MHz, DMSO-d₆): 2.97 (t, *J* = 6.6 Hz, 2H), 3.25 (s, 3H), 3.75 (s, 3H), 4.17 (dq, *J* = 1.5 Hz, *J* =4.5 Hz, 1H), 4.37 (m, 3H), 5.33 (dq, *J* = 1.5 Hz, *J* = 4.8 Hz, 1H), 7.32 (m, 1H), 7.51 (m, 2H).

### Step 7. Preparation of (5R)-3-(1-methyl-2-oxo-1,2,4,5-tetrahydro-3,1-benzoxazepin-7-yl)-2-oxo-1,3-oxazolidine-5-carboxamide.

To a mixture of methyl (5R)-3-(1-methyl-2-oxo-1,2,4,5-tetrahydro-3,1-benzoxazepin-7-yl)-2-oxo-1,3-oxazolidine-5-carboxylate (0.20 g, 0.62 mmol) in MeOH (5 mL) at 0°C is added NH₃ in MeOH solution (3 mL, 2.0 M, 6.0 mmol). The suspension is stirred at the same temperature for 2h and then concentrated. The crude product is recrystallized from MeOH/CH₂Cl₂ to afford the title compound. HPLC (SYMMETRY C₁₈ 3.5 µM, 4.6 x 30 mm column; gradient elution 2%-98% MeCN with 0.1% TFA over 6 min; 2 mL/min rate): retention time = 1.84 min; ¹H NMR (300 MHz, DMSO-d₆): 2.96 (t, *J* = 6.6 Hz, 2H), 3.25 (s, 3H), 4.01 (dd, *J* = 6.0 Hz, *J* = 9.3 Hz, 1H), 4.27 (t, *J* = 9.3 Hz, 1H), 4.35 (t, *J* = 6.6 Hz, 2H), 5.02 (dd, *J* = 6.3 Hz, *J* = 9.3 Hz, 1H), 7.31 (m, 1H), 7.52 (m, 2H), 7.61 (s, 1H), 7.85 (s, 1H); MS (m/z): [M+H]⁺ = 306.0.

### Example 19 Preparation of (5R)-N-methyl-3-(1-methyl-2-oxo-1,2,4,5-tetrahydro-3,1-benzoxazepin-7-yl)-2-oxo-1,3-oxazolidine-5-carboxamide.

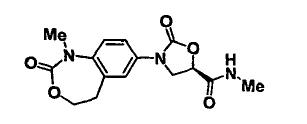

To a solution of methyl (5R)-3-(1-methyl-2-oxo-1,2,4,5-tetrahydro-3,1-benzoxazepin-7-yl)-2-oxo-1,3-oxazolidine-5-carboxylate (181 mg, 0.57 mmol) in MeOH (5 mL) at 0°C is added methylamine (1.2 mL of a 2.0 M solution in MeOH, 2.4 mmol). The suspension is stirred at the same temperature for 1 h and then concentrated to afford the title compound. HPLC (SYMMETRY C₁₈ 3.5 µM, 4.6 x 30 mm column; gradient elution 2%-98% MeCN with 0.1% TFA over 5 min; 2 mL/min rate): retention time = 1.89 min; ¹H NMR (300 MHz, DMSO-d₆): 2.65 (d, *J* = 4.5 Hz, 3H), 2.96 (t, *J* = 6.6 Hz, 2H), 3.26 (s, 3H), 4.02 (dd, *J* = 5.7 Hz, *J* = 9.0 Hz, 1H), 4.28 (t, *J* = 9.3 Hz, 1H), 4.35 (t, *J* = 6.6 Hz, 2H), 5.06 (dd, *J* = 5.7 Hz, *J* = 9.6 Hz, 1H), 7.32 (m, 1H), 7.53 (m, 2H), 8.38 (m, 1H); MS (m/z): [M+H]⁺ = 320.0.

### Example 20 Preparation of N-{[(5S)-3-(1-methyl-2-oxo-1,2,4,5-tetrahydro-3,1-benzoxazepin-7-yl)-2-oxo-1,3-oxazolidin-5-yl]methyl}acetamide.

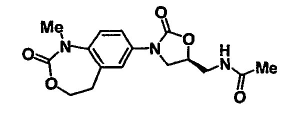

### Step 1. Preparation of tert-butyl (2R)-2-hydroxy-3-[(1-methyl-2-oxo-1,2,4,5-tetrahydro-3,1-benzoxazepin-7-yl)amino]propylcarbamate.

To a solution of 7-amino-1-methyl-4,5-dihydro-3,1-benzoxazepin-2(1H)-one (1.92 g, 10 mmol) in CH₃CN (40 mL) at 23°C is added LiOTf (1.72 g, 11 mmol), followed by tert-butyl (2S)-oxiran-2-ylmethylcarbamate (2.17 g, 12.5 mmol). After heating for 17 h at 70°C, the reaction mixture is cooled and concentrated. The residue is purified by chromatography on a silica gel column, eluting with a gradient increasing in polarity from 30% to 75% ethyl acetate-hexane. Relevant fractions are combined and concentrated to afford the title compound. ¹H NMR (300 MHz, DMSO-d₆): 1.37 (s, 9H), 2.79 (t, *J* = 6.6 Hz, 2H), 2.83-3.06 (m, 4H), 3.09 (s, 3H), 3.62 (m, 1H), 4.28 (t, *J* = 6.6 Hz, 2H), 4.87 (d, *J* = 4.8 Hz, 1H), 5.50 (m, 1H), 6.49 (m, 2H), 6.75 (m, 1H), 6.96 (d, *J* = 9.3 Hz, 1 H).

### Step 2. Preparation of tert-butyl-[(5S)-3-(1-methyl-2-oxo-1,2,4,5-tetrahydro-3,1-benzoxazepin-7-yl)-2-oxo-1,3-oxazolidin-5-yl]methylcarbamate.

To a solid mixture of tert-butyl (2R)-2-hydroxy-3-[(1-methyl-2-oxo-1,2,4,5-tetrahydro-3,1-benzoxazepin-7-yl)amino]propylcarbamate (1.58 g, 4.3 mmol) and 1,1'-carbonyldiimidazole (1.4 g, 8.6 mmol) at 23°C is added CH₃CN (20 mL). The resulting mixture is stirred at 65°C for 2 h, cooled to 23°C, and concentrated. The residue is taken into ethyl acetate (300 mL) and the organic solution washed with 0.5 M aqueous HCl, brine, and dried (Na₂SO₄), filtered, and concentrated. The residue is purified by chromatography on a silica gel column, eluting with a gradient increasing in polarity from 30% to 70% ethyl acetate in hexanes. Relevant fractions are combined and concentrated to afford the title compound. ¹H NMR (300 MHz, DMSO-d₆): 1.35 (s, 9H), 2.96 (t, *J* = 6.6 Hz, 2H), 3.25 (m, 5H), 3.79 (dd, *J* = 6.0 Hz, *J* = 9.0 Hz, 1H), 4.11 (t, J = 9.0 Hz, 1H), 4.35 (t, *J* = 6.6 Hz, 2H), 4.69 (m, 1H), 7.22 (m, 1H), 7.31 (d, *J* =8.4 Hz, 1H), 7.46 (m, 2H).

### Step 3. Preparation of N-{[(5S)-3-(1-methyl-2-oxo-1,2,4,5-tetrahydro-3,1-benzoxazepin-7-yl)-2-oxo-1,3-oxazolidin-5-yl]methyl}acetamide.

Trifluoroacetic acid (3 mL) is added to a solution of tert-butyl-[(5S)-3-(1-methyl-2-oxo-1,2,4,5-tetrahydro-3,1-benzoxazepin-7-yl)-2-oxo-1,3-oxazolidin-5-yl]methylcarbamate (313 mg, 0.81 mmol) in CH₂Cl₂ (10 mL) at 23°C. The reaction mixture is stirred at 23°C for 60 min and concentrated. The crude 7-[(5S)-5-(aminomethyl)-2-oxo-1,3-oxazolidin-3-yl]-1-methyl-4,5-dihydro-3,1-benzoxazepin-2(1H)-one obtained is directly used in the next step without further purification. Acetic anhydride (0.32 mL, 1.2 mmol) is added to a cooled (0°C) solution of 7-[(5S)-5-(aminomethyl)-2-oxo-1,3-oxazolidin-3-yl]-1-methyl-4,5-dihydro-3,1-benzoxazepin-2(1H)-one (0.81 mmol) in pyridine (5 mL). The reaction mixture is stirred at 23°C for 60 min, diluted with CH₂Cl₂ (300 mL), and washed with 1.0 M aqueous HCl, aqueous NaHCO₃ and dried (Na₂SO₄), filtered and concentrated. The crude product is purified by chromatography on a silica gel column, eluting with a gradient increasing in polarity from 0 to 5% methanol in methylene-chloride. Relevant fractions are combined and concentrated to afford the title compound. HPLC (SYMMETRY C₁₈ 3.5 µM, 4.6 x 30 mm column; gradient elution 2%-98% MeCN with 0.1% TFA over 6 min; 2 mL/min rate): retention time = 1.975 min; ¹H NMR (300 MHz, DMSO-d₆): 1.83 (s, 3H), 2.96 (t, *J* = 6.6 Hz, 2H), 3.25 (s, 3H), 3.41 (t, *J* = 5.7 Hz, 2H), 3.73 (dd, *J* = 6.3 Hz, *J* = 9.3 Hz, 1H), 4.11 (t, *J* = 9.0 Hz, 1H), 4.35 (t, *J* = 6.6 Hz, 2H), 4.72 (m, 1H), 7.30 (m, 1H), 7.47 (m, 2H), 8.23 (t, *J* = 5.7 Hz, 1H); MS (m/z): [M+H]⁺ = 334.3.

### Example 21 Preparation of N-{[(5S)-3-(1-methyl-2-oxo-1,2,4,5-tetrahydro-3,1-benzoxazepin-7-yl)-2-oxo-1,3-oxazolidin-5-yl]methyl}propanamide.

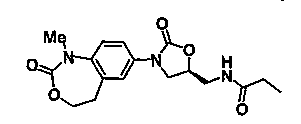

Trifluoroacetic acid (3 mL) is added to a solution of tert-butyl-[(5S)-3-(1-methyl-2-oxo-1,2,4,5-tetrahydro-3,1-benzoxazepin-7-yl)-2-oxo-1,3-oxazolidin-5-yl]methylcarbamate (313 mg, 0.81 mmol) in CH₂Cl₂ (10 mL) at 23°C. The reaction mixture is stirred at 23°C for 60 min and concentrated. The crude 7-[(5S)-5-(aminomethyl)-2-oxo-1,3-oxazolidin-3-yl]-1-methyl-4,5-dihydro-3,1-benzoxazepin-2(1H)-one obtained is directly used in the next step without further purification. Propionic anhydride (0.11 mL, 0.85 mmol) is added to cooled (0°C) solution of crude 7-[(5S)-5-(aminomethyl)-2-oxo-1,3-oxazolidin-3-yl]-1-methyl-4,5-dihydro-3,1-benzoxazepin-2(1H)-one (0.81 mmol) in pyridine (5 mL). The reaction mixture is stirred at 23°C for 60 min and diluted with CH₂Cl₂ (300 mL). The organic solution is washed with 1.0 M aqueous HCl, aqueous NaHCO₃, and dried (Na₂SO₄), filtered and concentrated. The crude product is purified by chromatography on a silica gel column, eluting with a gradient increasing in polarity from 0 to 5% methanol in methylene chloride. Relevant fractions are combined and concentrated to afford the title compound. ¹H NMR (300 MHz, DMSO-*d*₆): 0.95 (t, *J* = 7.8 Hz, 3H), 2.11 (q, *J* = 7.8 Hz, 2H), 2.96 (t, *J* = 6.6 Hz, 2H), 3.25 (s, 3H), 3.41 (t, *J* = 5.7 Hz, 2H), 3.74 (dd, *J* = 6.3 Hz, *J* = 9.0 Hz, 1H), 4.11 (t, *J* = 9.0 Hz, 1H), 4.35 (t, *J* = 6.6 Hz, 2H), 4.71 (m, 1H), 7.30 (m, 1H), 7.49 (m, 2H), 8.16 (t, *J* = 6.0 Hz, 1H); MS (m/z): [M+H]⁺ = 348.0.

### Example 22 Preparation of methyl [(5S)-3-(1-methyl-2-oxo-1,2,4,5-tetrahydro-3,1-benzoxazepin-7-yl)-2-oxo-1,3-oxazolidin-5-yl]methylcarbamate.

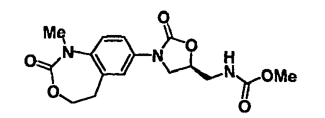

Trifluoroacetic acid (3 mL) is added to a solution of *tert*-butyl-[(5*S*)-3-(1-methyl-2-oxo-1,2,4,5-tetrahydro-3,1-benzoxazepin-7-yl)-2-oxo-1,3-oxazolidin-5-yl]methylcarbamate (313 mg, 0.81 mmol) in CH₂Cl₂ (10 mL) at 23°C. The reaction mixture is stirred at 23°C for 60 min and concentrated. The crude 7-[(5S)-5-(aminomethyl)-2-oxo-1,3-oxazolidin-3-yl]-1-methyl-4,5-dihydro-3,1-benzoxazepin-2(1H)-one obtained is directly used in the next step without further purification. Methyl chloroformate (0.5 mL, 6.47 mmol) is added to cooled (0°C) solution of 7-[(5*S*)-5-(aminomethyl)-2-oxo-1,3-oxazolidin-3-yl]-1-methyl-4,5-dihydro-3,1-benzoxazepin-2(1H)-one (0.81 mmol) in pyridine (5 mL). The reaction mixture is stirred at 23°C for 60 min and diluted with CH₂Cl₂ (300 mL). The organic solution is washed with 1.0 M aqueous HCl, aqueous NaHCO₃, and dried (Na₂SO₄), filtered and concentrated. The crude product is purified by chromatography on a silica gel column, eluting with a gradient increasing in polarity from 0 to 5% methanol in methylene chloride. Relevant fractions are combined and concentrated to afford the title compound. ¹H NMR (300 MHz, DMSO-d₆): 2.96 (t, *J* = 6.6 Hz, 2H), 3.25 (s, 3H), 3.38 (m, 2H), 3.53 (s, 3H), 3.77 (dd, *J* = 6.0 Hz, *J* = 9.0 Hz, 1H), 4.12 (t, *J* = 9.0 Hz, 1H), 4.35 (t, *J* = 6.6 Hz, 2H), 4.70 (m, 1H), 7.30 (m, 1H), 7.46-7.55 (m, 3H); MS (m/z): [M+H]⁺ = 350.0.

## Claims

1. The present invention provides a compound of formula I 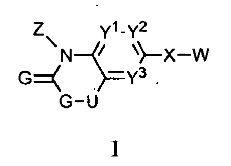 or a pharmaceutically acceptable salt thereof wherein:
X is a structure of the following formula i, ii, iii, or iv 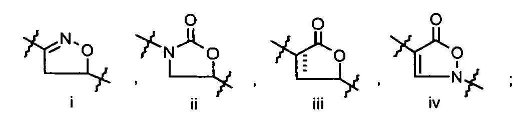
G is O, or S;
U is -(CR³R⁴)ₙ-, or CF₂;
W is
(a) CH₂NHC(=O)R¹,
(b) CH₂NHC(=S)R¹,
(c) CH₂NH-het,
(d) CH₂O-het,
(e) CH₂S-het,
(f) CH₂het,
(g) C(=O)NHR², or
(h) CH₂OH;
Y¹, Y² and Y³ are independently
(a) CH,
(b) N,
(c) N⁺-O⁻, or
(d) CF;
Z is C₁₋₄alkyl, optionally substituted with 1-3 fuloro;
R¹ is
(a) NH₂,
(b) NHC₁₋₆alkyl,
(c) C₁₋₆alkyl,
(b) C₂₋₆alkenyl,
(c) C₃₋₇cycloalkyl,
(e) (CH₂)ⱼC(=O)C₁₋₄alkyl,
(f) OC₁₋₆alkyl,
(g) SC₁₋₆alkyl, or
(h) (CH₂)ⱼC₃₋₇cycloalkyl;
R² is
(a) H,
(b) C₁₋₆alkyl,
(c) C₂₋₆alkenyl,
(d) C₃₋₇cycloalkyl, or
(e) -OC₁₋₄alkyl;
R³ and R⁴ are independently
(a) H
(b) C₁₋₆ alkyl, or
(c) R³ and R⁴ taken together with the carbon atom to which they attach form C₃₋₇cycloalkyl;
het is a five- (5) or six- (6) membered heterocyclic ring having 1-4 heteroatoms selected from the group consisting of oxygen, sulfur, and nitrogen within the ring, wherein each carbon atom in het is optionally substituted with C₁₋₆alkyl, C₂₋₆alkenyl, C₂₋₆alkynyl, C₃₋₇cycloalkyl , halo, OR⁵, SR⁵, CN, NO₂ NR⁵R⁶, oxo, CF₃, OCF₃, C(=O)C₁₋₄alkyl, OC(=O)C₁₋₄alkyl, C(=O)OR⁵, or S(O)ₘC₁₋₄alkyl; at each occurrence, C₁₋₆alkyl, C₂₋₆alkenyl, C₂₋₆alkynyl, or C₃₋₇cyctoalkyl is optionally substituted with 1-3 halo, OR⁵, SR⁵, CN, N₃, NO₂, NR⁵R⁶, G(=O)C₁₋₄alkyl, OC(=O)C₁₋₄alkyl, C(=O)OC₁₋₄alkyl, or S(O)ₘC₁₋₄alkyl, wherein R⁵ and R⁶ are independently H, or C₁₋₄alkyl, optionally substituted with OH, phenyl, or OC₁₋₄alkyl;
each n is independently 1 or 2;
m is 0, 1, or 2; and
each j is independently 0-4.

2. A compound of claim 1 wherein X is a structure of formula ii

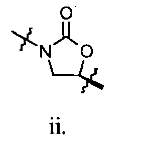

3. A compound of claim 2 wherein W is CH₂NHC(=O)R¹; R¹ is C₁₋₄alkyl, or OC₁₋₄alkyl; wherein the alkyl is optionally substituted with F, or Cl.

4. A compound of claim 3 wherein R¹ is CH₃, CH₂CH₂, or OCH₃.

5. A compound of claim 2 wherein W is 1,2,3-triazole-1-yl methyl.

6. A compound of claim 2 wherein W is C(=O)NHR²; R² is C₁₋₄alkyl, or OC₁₋₄alkyl; wherein the alkyl is optionally substituted with F, or Cl.

7. A compound of claim 6 wherein R² is H, CH₃, or OCH₃.

8. A compound of claim 2 wherein U is CH₂, or CH₂CH₂.

9. A compound of claim 2 which is a compound of formula II 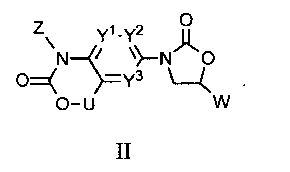 wherein U is CH₂, or CH₂CH₂; W is CH₂NHC(=O)R¹, CH₂het, or C(=O)NHR²; Y¹, Y² and Y³ are independently CH, or CF; R¹ is C₁₋₄alkyl, or OC₁₋₄alkyl; R² is H, C₁₋₄alkyl, or -OC₁₋₄alkyl; and Z is CH₃.

10. A compound of claim 9 wherein R¹ is CH₃, CH₂CH₂, or OCH₃; R² is H, CH₃, or OCH₃.

11. A compound of claim 2 which is
(1) (S)-N-((3-(1-methyl-2-oxo-2,4-dihydro-1H-benzo[d][1,3]oxazin-6-yl)-2-oxooxazolidin-5-yl)methyl)acetamide,
(2) (*S*)-*N*-((3-(1-methyl-2-oxo-2,4-dihydro-1H-benzo[*d*][1,3]oxazin-6-yl)-2-oxooxazolidin-5-yl)methyl)propionamide,
(3) (*S*)-methyl (3-(1-methyl-2-oxo-2,4-dihydro-1H-benzo[d][1,3]oxazin-6-yl)-2-oxooxazolidin-5-yl)methylcarbamate,
(4) (*R*)-3-(1-methyl-2-oxo-2,4-dihydro-1H-benzo[d][1,3]oxazin-6-yl)-2-oxooxazolidine-5-carboxamide,
(5) (*R*)-*N*-methyl-3-(1-methyl-2-oxo-2,4-dihydro-1H-benzo[d][1,3]oxazin-6-yl)-2-oxooxazolidine-5-carboxamide,
(6) *(S*)-fuoromethyl (3-(1-methyl-2-oxo-2,4-dihydro-1H-benzo[d][1,3]oxazin-6-yl)-2-oxooxazolidin-5-yl)methylcarbamate,
(7) (*R*)-6-(5-((1H-1,2,3-triazol-1-yl)methyl)-2-oxooxazolidin-3-yl)-1-methyl-1H-benzo[d][1,3]oxazin-2(4H)-one,
(8) (*R*)-3-(8-fluoro-1-methyl-2-oxo-2,4-dihydro-1H-benzo[d][1,3]oxazin-6-yl)-2-oxooxazolidine-5-carboxamide,
(9) (*R*)-3-(8-fluoro-1-methyl-2-oxo-2,4-dihydro-1H-benzo[d][1,3]oxazin-6-yl)-N-methyl-2-oxooxazolidine-5-carboxamide,
(10) (*S*)-*N*-((3-(8-fluoro-1-methyl-2-oxo-2,4-dihydro-1H-benzo[d][1,3]oxazin-6-yl)-2-oxooxazolidin-5-yl)methyl)acetamide,
(11) (*S*)-*N*-((3-(8-fluoro-1-methyl-2-oxo-2,4-dihydro-1H-benzo[d][1,3]oxazin-6-yl)-2-oxooxazolidin-5-yl)methyl)propionamide,
(12) (*S*)-methyl (3-(8-fluoro-1-methyl-2-oxo-2,4-dihydro-1H-benzo[d][1,3]oxazin-6-yl)-2-oxooxazolidin-5-yl)methylcarbamate,
(13) (*S*)-*N*-((3-(5,8-difluoro-1-methyl-2-oxo-2,4-dihydro-1H-benzo[d][1,3]oxazin-6-yl)-2-oxooxazolidin-5-yl)methyl)acetamide,
(14) (*S*)-methyl (3-(5,8-difluoro-1-methyl-2-oxo-2,4-dihydro-1H-benzo[d][1,3]oxazin-6-yl)-2-oxooxazolidin-5-yl)methylcarbamate,
(15) *N-*{[(5*S*)-3-(9-Fluoro-1-methyl-2-oxo-1,2,4,5-tetrahydro-3,1-benzoxazepin-7-yl)-2-oxo-1,3-oxazolidin-5-yl]methyl}acetamide,
(16) *N-*{[(5*S*)-3-(9-fluoro-1-methyl-2-oxo-1,2,4,5-tetrahydro-3,1-benzoxazepin-7-yl)-2-oxo-1,3-oxazolidin-5-yl]methyl}propanamide,
(17) methyl [(5*S*)-3-(9-fluoro-1-methyl-2-oxo-1,2,4,5-tetrahydro-3,1-benzoxazepin-7-yl)-2-oxo-1,3-oxazolidin-5-yl]methylcarbamate,
(18) (5*R*)-3-(1-methyl-2-oxo-1,2,4,5-tetrahydro-3,1-benzoxazepin-7-yl)-2-oxo-1,3-oxazolidine-5-carboxamide,
(19) (5*R*)-*N*-methyl-3-(1-methyl-2-oxo-1,2,4,5-tetrahydro-3,1-benzoxazepin-7-yl)-2-oxo-1,3-oxazolidine-5-carboxamide,
(20) *N*-{[(5*S*)-3-(1-methyl-2-oxo-1,2,4,5-tetrahydro-3,1-benzoxazepin-7-yl)-2-oxo-1,3-oxazolidin-5-yl]methyl}acetamide,
(21) *N-*{[(5*S*)-3-(1-methyl-2-oxo-1,2,4,5-tetrahydro-3,1-benzoxazepin-7-yl)-2-oxo-1,3-oxazolidin-5-yl]methyl}propanamide, or
(22) methyl [(5*S*)-3-(1-methyl-2-oxo-1,2,4,5-tetrahydro-3,1-benzoxazepin-7-yl)-2-oxo-1,3-oxazolidin-5-yl]methylcarbamate.

12. A pharmaceutical composition comprising a compound of claim 1 or a pharmaceutically acceptable salt thereof, and a pharmaceutically acceptable carrier.

13. A compound of claim 1 for use in therapy.

14. A compound of claim 1 for treating bacteria infections.

15. Use of a compound of claim 1 for the manufacture of a medicament for treating bacteria infections.

## Patentansprüche

1. Die vorliegende Erfindung stellt eine Verbindung der Formel I 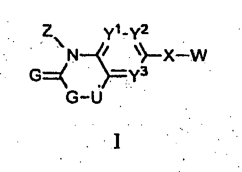 oder ein pharmazeutisch verträgliches Salz davon bereit, worin:
X eine Struktur der folgenden Formel i, ii, iii oder iv ist 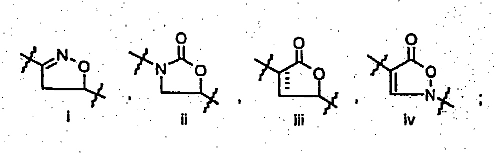
G O oder S ist;
U -(CR³R⁴)ₙ- oder CF₂ ist;
W
(a) CH₂NHC(=O)R¹,
(b) CH₂NHC(=S)R¹,
(c) CH₂NH-Het,
(d) CH₂OHet,
(e) CH₂S-Het,
(f) CH₂Het,
(g) C(=O)NHR² oder
(h) CH₂OH ist;
Y¹, Y² und Y³ unabhängig
(a) CH,
(b) N,
(c) N⁺-O⁻ oder
(d) CF sind;
Z C₁₋₄Alkyl ist, gegebenenfalls substituiert mit 1-3 Fluor;
R¹
(a) NH₂,
(b) NHC₁₋₆Alkyl,
(c) C₁₋₆Alkyl,
(b) C₂-₆Alkenyl,
(c) C₃₋₇Cycloalkyl,
(e) (CH₂)ⱼC(=O)C₁₋₄Alkyl,
(f) OC₁₋₆Alkyl,
(g) SC₁₋₆Alkyloder
(h) (CH₂)ⱼC₃₋₇Cycloalkyl ist;
R²
(a) H,
(b) C₁₋₆Alkyl,
(c) C₂₋₆Alkenyl,
(d) G₃₋₇Cycloalkyl oder
(e) -OC₁₋₉Alkyl ist;
R³ und R⁴ unabhängig
(a) H,
(b) C₁₋₆Alkyl sind oder
(c) R³ und R⁴ zusammengenommen mit dem Kohlenstoffatom, an das sie binden, C₃₋₇Cycloalkyl bilden;
Het ein fünf- (5) oder sechs- (6) gliedriger heterocyclischer Ring mit 1-4 Heteroatomen, ausgewählt aus der Gruppe, bestehend aus Sauerstoff, Schwefel und Stickstoff innerhalb des Rings, ist, wobei jedes Kohlenstoffatom in Het gegebenenfalls substituiert ist mit C₁₋₆Alkyl, C₂-₆Alkenyl, C₂₋₆Alkinyl, C₃₋₇Cycloalkyl, Halogen, OR⁵, SR⁵, CN, NO₂, NR⁵R⁶, OXO, CF₃, OCF₃, C(=O)C₁₋₄Alkyl, OC(=O)C₁₋₄Alkyl, C(=O)OR⁵ oder S(O)ₘC₁₋₄Alkyl; wobei bei jedem Vorkommen C₁₋₆Alkyl, C₂₋₆Alkenyl, C₂₋₆Alkinyl oder C₃₋₇Cycloalkylgegebenenfalls substituiert ist mit 1-3 Halogen, OR⁵, SR⁵, CN, N₃⁻, NO₂, NR⁵R⁶, C(=O)C₁₋₄Alkyl, OC(=O)C₁₋₄Alkyl, C(=O)OC₁₋₄Alkyl oder S(O)C₁₋₄Alkyl, wobei R⁵ und R⁶ unabhängig H oder C₁₋₄Alkyl sind, gegebenenfalls substituiert mit OH, Phenyl oder OC₁₋₄Alkyl;
jedes n unabhängig 1 oder 2 ist;
m 0, 1 oder 2 ist; und
jedes j unabhängig 0-4 ist.

2. Verbindung nach Anspruch 1, worin X eine Struktur der Formel ii ist

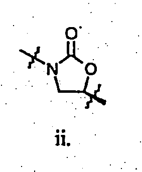

3. Verbindung nach Anspruch 2, worin W CH₂NHC(=O)R¹ ist; R¹ C₁₋₄Alkyl oder OC₁₋₄Alkyl ist, wobei das Alkyl gegebenenfalls substituiert ist mit F oder Cl.

4. Verbindung nach Anspruch 3, worin R¹ CH₃, CH₂CH₂ oder OCH₃ ist.

5. Verbindung nach Anspruch 2, worin W 1,2,3-Triazol-1-yl-methyl ist.

6. Verbindung nach Anspruch 2, worin W C(=O)NHR² ist; R² C₁₋₄Alkyl oder OC₁₋₄Alkyl ist, wobei das Alkyl gegebenenfalls substituiert ist mit F oder Cl.

7. Verbindung nach Anspruch 6, worin R² H, CH₃ oder OCH₃ ist.

8. Verbindung nach Anspruch 2, worin U CH₂ oder CH₂CH₂ ist.

9. Verbindung nach Anspruch 2, welche eine Verbindung der Formel II ist 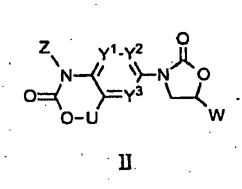 worin U CH₂ oder CH₂CH₂ ist; W CH₂NHC(=O)R¹, CH₂Het oder C(=O)NHR² ist; Y¹, Y² und Y³ unabhängig CH oder CF sind; R¹ C₁₋₄Alkyl oder OC₁₋₄Alkyl ist; R² H, C₁₋₄Alkyl oder -OC₁₋₄Alkyl ist; und Z CH₃ ist.

10. Verbindung nach Anspruch 9, worin R¹ CH₃, CH₂CH₂ oder OCH₃ ist; R² H, CH₃ oder OCH₃ ist.

11. Verbindung nach Anspruch 2, welche ist.
(1) (S)-N-((3-(1-Methyl-2-oxo-2,4-dihydro-1H-benzo[d][1,3]oxazin-6-yl)-2-oxooxazolidin-5-yl)methyl)acetamid,
(2) (S)-N-((3-(1-Methyl-2-oxo-2,4-dihydro-1H-benzo[d][1,3]oxazin=6-yl)-2-oxooxazplidin-5-yl)methyl)propionamid,
(3) (S)-Methyl-(3-(1-methyl-2-oxo-2,4-dihydro-1H-benzo[d][1,3]oxazin-6-yl)-2-oxooxazolidin-5-yl)methylcarbamat,
(4) (R)-3-(1-Methyl-2-oxo-2,4-dihydro-1H-benzo[d][1,3]oxazin-6-yl)-2-oxooxazolidin-5-carboxamid,
(5) (R)-N-Methyl-3-(1-methyl-2-oxo-2,4-dihydro-1H-benzo[d][1,3]oxazin-6-yl)-2-oxooxazolidin-5-carboxamid,
(6) (S)-Fluormethyl-(3-(1-methyl-2-oxo-2,4-dihydro-1H-benzo[d][1,3]oxazin-6-yl)-2-oxooxazolidin-5-yl)methylcarbamat,
(7) (R)-6-(5-((1H-1,2,3-Triazol-1-yl)methyl)-2-oxooxazolidin-3-yl)-1-methyl-1H-benzo[d][1,3]oxazin-2(4H)-on,
(8) (R)-3-(8-Fluor-1-methyl-2-oxo-2,4-dihydro-1H-benzo[d][1,3]oxazin-6-yl)-2-oxooxazolidin-5-carboxamid,
(9) (R)-3-(8-Fluor-1-methyl-2-oxo-2,4-dihydro-1H-benzo[d][1,3]oxazin-6-yl)-N-methyl-2-oxooxazolidin-5-carboxamid,
(10) (S)-N-((3-(8-Fluor-1-methyl-2-oxo-2,4-dihydro-1H-benzo[d][1,3]oxazin-6-yl)-2-oxooxazolidin-5-yl)methyl)acetamid,
(11) (S)-N-((3-(8-Fluor-1-methyl-2-oxo-2,4-dihydro-1H-benzo[d][1,3]oxazin-6-yl) -2-oxooxazolidin-5-yl)methyl)propionamid,
(12) (S)-Methyl-(3-(8-fluor-1-methyl-2-oxo-2,4-dihydro-1H-benzo[d][1,3]oxazin-6-yl)-2-oxooxazolidin-5-yl)methylcarbamat,
(13) (S)-N-((3-(5,8-Difluor-1-methyl-2-oxo-2,4-dihydro-1H-benzo[d][1,3]oxazin-6-yl)-2-oxooxazolidin-5-yl)methyl)acetamid,
(14) (S)-Methyl-(3-(5,8-diflüor-1-methyl-2-oxo-2,4-dihydro-1H-benzo[d][1,3]oxazin-6-yl)-2-oxooxazolidin-5-yl)methylcarbamat,
(15) N-{[(5S)-3-(9-Fluor-1-methyl-2-oxo-1,2,4,5-tetrahydro-3,1-benzoxazepin-7-yl)-2-oxo-1,3-oxazolidin-5-yl]methyl}acetamid,
(16) N-{[(5S)-3-(9-Fluor-1-methyl-2-oxo-1,2,4,5-tetrahydro-3,1-benzoxazepin-7-yl)-2-oxo-1,3-oxazolidin-5-yl]methyl}propanamid,
(17) Methyl-[(5S)-3-(9-fluor-1-methyl-2-oxo-1,2,4,5-tetrahydro-3,1-benzoxazepin-7-yl)-2-oxo-1,3-oxazolidin-5-yl]methylcarbamat,
(18) (5R)-3-(1-Methyl-2-oxo-1,2,4,5-tetrahydro-3,1-benzoxazepin-7-yl)-2-oxo-1,3-oxazolidin-5-carboxamid,
(19) (5R)-N-Methyl-3-(1-methyl-2-oxo-1,2,4,5-tetrahydro-3,1-benzoxazepin-7-yl)-2-oxo-1,3-oxazolidin-5-carboxamid,
(20) N-{[(5S)-3-(1-Methyl-2-oxo-1,2,4,5-tetrahydro-3,1-benzoxazepin-7-yl)-2-oxo-1,3-oxazolidin-5-yl]methyl}acetamid,
(21) N-{[(5S)-3-(1-Methyl-2-oxo-1,2,4,5-tetrahydro-3,1-benzoxazepin-7-yl)-2-oxo-1,3-oxazolidin-5-yl]methyl}propanamid oder
(22) Methyl-[(5S)-3-(1-methyl-2-oxo-1,2,4,5-tetrahydro-3,1-benzoxazepin-7-yl)-2-oxo-1,3-oxazolidin-5-yl]methylcarbamat.

12. Pharmazeutische Zusammensetzung, umfassend eine Verbindung nach Anspruch 1 oder ein pharmazeutisch verträgliches Salz davon und einen pharmazeutisch verträglichen Träger.

13. Verbindung nach Anspruch 1 zur Verwendung in der Therapie.

14. Verbindung nach Anspruch 1 zum Behandeln von Bakterieninfektionen.

15. Verwendung einer Verbindung nach Anspruch 1 zur Herstellung eines Medikaments zum Behandeln von Bakterieninfektionen.

## Revendications

1. Composé de formule 1 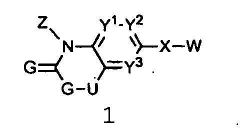 ou un de ses sels pharmaceutiquement acceptables, formule dans laquelle :
X représente une structure répondant à la formule i, ii, iii ou iv suivante 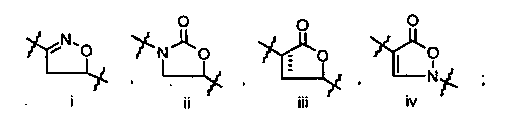
G représente O ou S ;
U représente un groupe -(CR³R⁴)ₙ- ou CF₂ ;
W représente un groupe
(a) CH₂NHC(=O)R¹,
(b) CH₂NHC(=S)R¹,
(c) CH₂NH-het,
(d) CH₂O-het ,
(e) C₂S-het,
(f) CH₂het,
(g) C(=O)NHR², ou
(h) CH₂OH ;
Y¹, Y² et Y³ représentent indépendamment un groupe
(a) CH,
(b) N,
(c) N⁺-O⁻, ou
(d) CF ;
Z représente un groupe alkyle en C₁ à C₄, facultativement substitué avec 1 à 3 substituants fluoro ;
R¹ représente un groupe
(a) NH₂,
(b) NH(alkyle en C₁ à C₆),
(c) alkyle en C₁ à C₆,
(b) alcényle en C₂ à C₆,
(c) cycloalkyle en C₃ à C₇,
(e) (CH₂)ⱼC(=O) (alkyle en C₁ à C₄),
(f) O(alkyle en C₁ à C₆),
(g) S(alkyle en C₁ à C₆), ou
(h) (CH₂)ⱼ(cycloalkyle en C₃ à C₇) ;
R² représente un groupe
(a) H,
(b) alkyle en C₁ à C₆,
(c) alcényle en C₂ à C₆,
(d) cycloalkyl en C₃ à C₇, ou
(e) O(alkyle en C₁ à C₄) ;
R³ et R⁴ représentent indépendamment
(a) H
(b) un groupe alkyle en C₁ à C₆, ou
(c) R³ et R⁴, pris conjointement avec l'atome de carbone auquel ils sont fixés, forment un groupe cycloalkyle en C₃ à C₇ ;
het représente un noyau hétérocyclique pentagonal (à cinq membres) ou hexagonal (à six membres) comprenant 1 à 4 hétéroatomes choisis dans le groupe consistant en l'oxygène, le soufre et l'azote dans le noyau, dans lequel chaque atome de carbone dans het est facultativement substitué avec un substituant alkyle en C₁ à C₆, alcényle en C₂ à C₆, alcynyle en C₂ à C₆, cycloalkyle en C₃ à C₇, halogéno, OR⁵, SR⁵, CN, NO₂, NR⁵R⁶, oxo, CF₃, OCF₃, C(=O)(alkyle en C₁ à C₄), OC(=O) (alkyle en C₁ à C₄), C(=O)OR⁵, ou S(O)ₘ(alkyle en C₁ à C₄) ; à chaque occurrence, le groupe alkyle en C₁ à C₆, alcényle en C₂ à C₆, alcynyle en C₂ à C₆ ou cycloalkyle en C₃ à C₇ est facultativement substitué avec 1 à 3 substituants halogéno, OR⁵, SR⁵, CN, N₃, NO₂, NR⁵R⁶, C(=O)(alkyle en C₁ à C₄), OC(=O)(alkyle en C₁ à C₄), C(=O)O(alkyle en C₁ à C₄) ou S(O)ₘ(alkyle en C₁ à C₄), dans lesquels R⁵ et R⁶ représentent indépendamment H ou un groupe alkyle en C₁ à C₄, facultativement substitué avec un substituant OH, phényle ou O(alkyle en C₁ à C₄) ;
chaque indice n est indépendamment égal à 1 ou 2 ;
m est égal à 0, 1 ou 2 ; et
chaque indice j a indépendamment une valeur de 0 à 4.

2. Composé suivant la revendication 1, dans lequel X représente une structure de formule ii

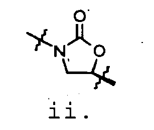

3. Composé suivant la revendication 2, dans lequel W représente un groupe CH₂NHC(=O)R¹ ; R¹ représente un groupe alkyle en C₁ à C₄ ou O(alkyle en C₁ à C₄) ; dans lequel le groupe alkyle est facultativement substitué avec un substituant F ou Cl.

4. Composé suivant la revendication 3, dans lequel R¹ représente un groupe CH₃, CH₂CH₂ ou OCH₃.

5. Composé suivant la revendication 2, dans lequel W représente un groupe 1,2,3-triazole-1-ylméthyle.

6. Composé suivant la revendication 2, dans lequel W représente un groupe C(=O)NHR² ; R² représente un groupe alkyle en C₁ à C₄ ou O(alkyle en C₁ à C₄) ; dans lequel le groupe alkyle est facultativement substitué avec un substituant F ou Cl.

7. Composé suivant la revendication 6, dans lequel R² représente H, un groupe CH₃ ou OCH₃.

8. Composé suivant la revendication 2, dans lequel U représente un groupe CH₂ ou CH₂CH₂.

9. Composé suivant la revendication 2, qui est un composé de formule II 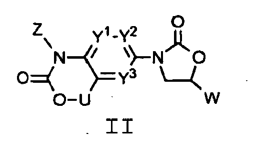 dans laquelle U représente un groupe CH₂ ou CH₂CH₂ ; W représente un groupe CH₂NHC(=O)R¹, CH₂het ou C(=O)NHR² ; Y¹, Y² et Y³ représentent indépendamment un groupe CH ou CF ; R¹ représente un groupe alkyle en C₁ à C₄ ; R² représente H, un groupe alkyle en C₁ à C₄ ou -O(alkyle en C₁ à C₄) ; et Z représente un groupe CH₃.

10. Composé suivant la revendication 9, dans lequel R¹ représente un groupe CH₃, CH₂CH₂ ou OCH₃ ; R² représente H ou un groupe CH₃ ou OCH₃.

11. Composé suivant la revendication 2, qui est
(1) le (S)-N-((3-(1-méthyl-2-oxo-2,4-dihydro-1H-benzo-[d][1,3]oxazine-6-yl)-2-oxooxazolidine-5-yl)méthyl)acétamide,
(2) le (*S*)-*N*-((3-(1-méthyl-2-oxo-2,4-dihydro-1H-benzo-[*d*][1,3]oxazine-6-yl)-2-oxooxazolidine-5-yl)méthyl)propionamide,
(3) le (3-(1-méthyl-2-oxo-2,4-dihydro-1H-benzo[d][1,3]-oxazine-6-yl)-2-oxooxazolidine-5-yl)méthylcarbamate de (*S*)-méthyle,
(4) le (*R*)-3-(1-méthyl-2-oxo-2,4-dihydro-1H-benzo-[d]-[1,3]oxazine-6-yl)-2-oxooxazolidine-5-carboxamide,
(5) le (*R*)-*N*-méthyl-3-(1-méthyl-2-oxo-2,4-dihydro-1H-benzo-[d][1,3]oxazine-6-yl)-2-oxooxazolidine-5-carboxamide,
(6) le (3-(1-méthyl-2-oxo-2,4-dihydro-1H-benzo[d][1,3]-oxazine-6-yl)-2-oxooxazolidine-5-yl)méthylcarbamate de (*S*)-fluorométhyle,
(7) la (*R*)-6-(5-((1H-1,2,3-triazole-1-yl)méthyl)-2-oxo-oxazolidine-3-yl)-1-méthyl-1H-benzo[d][1,3]oxazine-2(4H)-one,
(8) le (*R*)-3-(8-fluoro-1-méthyl-2-oxo-2,4-dihydro-1H-benzo[d][1,3]oxazine-6-yl)-2-oxooxazolidine-5-carboxamide,
(9) le (*R*)-3-(8-fluoro-1-méthyl-2-oxo-2,4-dihydro-1H-benzo[d][1,3]oxazine-6-yl)-N-méthyl-2-oxooxazolidine-5-carboxamide,
(10) le (*S*)-*N*-((3-(8-fluoro-1-méthyl-2-oxo-2,4-dihydro-1H-benzo[d] [1,3]oxazine-6-yl)-2-oxooxazolidine-5-yl)méthyl)-acétamide,
(11) le (*S*)*-N*-((3-(8-fluoro-1-méthyl-2-oxo-2,4-dihydro-1H-benzo[d] [1,3]oxazine-6-yl)-2-oxooxazolidine-5-yl)méthyl)-propionamide,
(12) le 3-(8-fluoro-1-méthyl-2-oxo-2,4-dihydro-1H-benzo-[d][1,3]oxazine-6-yl)-2-oxooxazolidine-5-yl)méthylcarbamate de (*S*)-méthyle,
(13) le (*S*)-*N*-((3-(5,8-difluoro-1-méthyl-2-oxo-2,4-dihydro-1H-benzo[d][1,3]oxazine-6-yl)-2-oxooxazolidine-5-yl)méthyl)-acétamide,
(14) le 3-(5,8-difluoro-1-méthyl-2-oxo-2,4-dihydro-1H-benzo[d][1,3]oxazine-6-yl)-2-oxooxazolidine-5-yl)méthylcarbamate de (*S*)-méthyle,
(15) le *N*-{[(5*S*)-3-(9-fluoro-1-méthyl-2-oxo-1,2,4,5-tétrahidro-3,1-benzoxazépine-7-yl)-2-oxo-1,3-oxazolidine-5-yl]méthyl}acétamide ;
(16) le *N*-{[(5*S*)-3-(9-fluoro-1-méthyl-2-oxo-1,2,4,5-tétrahydro-3,1-benzoxazépine-7-yl)-2-oxo-1,3-oxazolidine-5-yl]méthyl}propanamide,
(17) le [(5*S*)-3-(9-fluoro-1-méthyl-2-oxo-1,2,4,5-tétrahydro-3,1-benzoxazépine-7-yl)-2-oxo-1,3-oxazolidine-5-yl]méthylcarbamate de méthyle,
(18) le (5*R*)-3-(1-méthyl-2-oxo-1,2,4,5-tétrahydro-3,1-benzoxazépine-7-yl)-2-oxo-1,3-oxazolidine-5-carboxamide,
(19) le (5*R*)-*N*-méthyl-3-(1-méthyl-2-oxo-1,2,4,5-tétrahydro-3,1-benzoxazépine-7-yl)-2-oxo-1,3-oxazolidine-5-carboxamide,
(20) le *N*-{[(5*S*)-3-(1-méthyl-2-oxo-1,2,4,5-tétrahydro-3,1-benzoxazépine-7-yl)-2-oxo-1,3-oxazolidine-5-yl]méthyl}-acétamide ;
(21) le *N*-{[(5*S*)-3-(1-méthyl-2-oxo-1,2,4,5-tétrahydro-3,1-benzoxazépine-7-yl)-2-oxo-1,3-oxazolidine-5-yl]méthyl}-propanamide, ou
(22) le (5*S*)-3-(1-méthyl-2-oxo-1,2,4,5-tétrahydro-3,1-benzoxazépine-7-yl)-2-oxo-1,3-oxazolidine-5-yl]méthylcarbamate de méthyle.

12. Composition pharmaceutique comprenant un composé de la revendication 1 ou un de ses sels pharmaceutiquement acceptables et un support pharmaceutiquement acceptable.

13. Composé suivant la revendication 1, destiné à être utilisé en thérapie.

14. Composé suivant la revendication 1, destiné au traitement d'infections bactériennes.

15. Utilisation d'un composé de la revendication 1 pour la production d'un médicament destiné au traitement d'infections bactériennes.
